# EUROPÄISCHE PATENTSCHRIFT

(11) **EP 3 942 013 B1**
(45) Veröffentlichungstag und Bekanntmachung des Hinweises auf die Patenterteilung: **06.05.2026**
(21) Anmeldenummer: 20714162.3
(22) Anmeldetag: 19.03.2020
(51) Int. Cl.: C12M 1/00, C12M 1/34

(54) **BEHÄLTER ZUR AUFBEWAHRUNG, MISCHUNG UND/ODER KULTIVIERUNG EINES MEDIUMS**
CONTAINER FOR STORING, MIXING AND/OR CULTIVATING A MEDIUM
RÉCIPIENT POUR LE STOCKAGE, LE MÉLANGE ET/OU LA CULTURE D'UN MILIEU

(30) Priorität: 21.03.2019 DE 102019001995
(43) Veröffentlichungstag der Anmeldung: 26.01.2022
(73) Patentinhaber: Sartorius Stedim Biotech GmbH, 37079 Göttingen (DE)
(72) Erfinder: GRIMM, Christian, 37308 Heilbad Heiligenstadt (DE); PREDIGER, Andreas, 37073 Göttingen (DE); BODE, Matthias, 37075 Göttingen (DE); PAJAK, Arkadiusz, Chippenham Wiltshire SN14 0Qt (GB)
(74) Vertreter: Novagraaf International SA
(86) Internationale Anmeldenummer: PCT/EP2020/057633
(87) Internationale Veröffentlichungsnummer: WO 2020/188037

(56) Entgegenhaltungen:
- DE-U1- 202018 104 472
- US-A1- 2011 201 100

## Beschreibung

Die Erfindung betrifft einen Behälter zur Aufbewahrung, Mischung und/oder Kultivierung eines Mediums, insbesondere einen Bioreaktor für ein Medium, mit einem Leitungssystem, einen Leitungskörper eines solchen Leitungssystems sowie ein Messsystem für einen Behälter zur Aufbewahrung, Mischung und/oder Kultivierung eines Mediums, insbesondere einen Bioreaktor.

Bioreaktoren finden beispielsweise in Brauereien, Winzereien und der Pharma- und Kosmetikindustrie zur Kultivierung von Medien Anwendung. Solche Medien können in Einwegbeuteln oder in Mehrwegbehältnissen bereitgestellt werden, die ein Volumen von mehreren hundert Litern umfassen können. Die biologischen Medien werden in einen Behälter (z.B. Bioreaktor) eingebracht und über einen vorbestimmten Zeitraum von üblicherweise mehreren Stunden Dauer auf einer vorbestimmbaren Temperatur temperiert und gegebenenfalls unter Zufuhr von Sauerstoff kultiviert. Von großer Bedeutung ist eine zuverlässige Kontrolle des Kultivierungsprozesses, insbesondere mittels Vornehmen unterschiedlicher Messungen an dem biologischen Medium. Da die Handhabung eines Bioreaktors üblicherweise in einer sterilen Umgebung erfolgt, ergeben sich besonders hohe Anforderungen an die Durchführung solcher Messungen bzw. an die verwendeten Messsysteme, um die Qualitätssicherung des Mediums zu gewährleisten. Auch ist es vorteilhaft, wenn die mit den Medien in Kontakt kommenden Elemente des Messsystems sterilisierbar sind.

Häufig werden Messungen des Mediums von verschiedenen Messkomponenten in einem Leitungssystems durchgeführt, welches das zu kultivierende Medium aus dem Behälter, wie z.B. einem Bioreaktor, abführt. In der Regel werden dafür die verschiedenen und mehrfach verwendbaren Messkomponenten an ein Einweg-Leitungssystem ("Single-Use"-Leitungssystem) angeschlossen. Messsysteme für solche Single-Use-Leitungssysteme umfassen daher in der Regel mehrere einzelne Messkomponenten zum Messen von bestimmten Parametern eines Mediums. Das Einbinden dieser Vielzahl von Messkomponenten in das Single-Use-Leitungssystem erfolgt durch Anschließen der individuellen Sensorelemente an separate bzw. externe Leitungskomponenten des Leitungssystems. Jedoch birgt dieses Vorgehen ein erhöhtes Risiko von Leckagen, insbesondere durch unsachgemäße Montage der einzelnen Verbindungsstellen. Auch ist das Anschließen sowie das vorherige Sterilisieren der einzelnen Messkomponenten arbeits- und kostenintensiv. Darüber hinaus kann ein bereits an das Leitungssystem angeschlossene Messkomponente, beispielsweise bei einem Defekt, nur unter großem Aufwand ausgetauscht werden.

US 2011/201100 A1 beschreibt einen Einweg-Kanal, an welchen modulare Sensoren zum Messen von physikalischen Variablen und anderen Parametern eines in einem Bioreaktor befindlichen Mediums angeschlossen werden können. DE 20 2018 104482 U1 beschreibt eine Vorrichtungsanordnung mit einer Messeinrichtung zum Messen eines multivariaten Parameters eines Mediums.

Es ist daher eine Aufgabe der vorliegenden Erfindung, einen Behälter zur Aufbewahrung, Mischung und/oder Kultivierung eines Mediums, insbesondere einen Bioreaktor für ein Medium, mit einem Leitungssystem sowie ein Messsystem für einen Behälter zur Aufbewahrung, Mischung und/oder Kultivierung eines Mediums, insbesondere einen Bioreaktor, bereitzustellen, welcher/s eine hohe Variabilität an Messungen erlaubt und gleichzeitig die Prozesssicherheit erhöht.

Die genannte Aufgabe wird durch die Gegenstände der unabhängigen Ansprüche gelöst. Vorteilhafte Ausführungsformen sind Gegenstand der Unteransprüche.

Insbesondere stellt die vorliegende Erfindung einen Leitungskörper für ein Leitungssystem eines Behälters zur Aufbewahrung, Mischung und/oder Kultivierung eines Mediums, insbesondere eines Bioreaktors, bereit, welcher einfach zu Handhaben ist, um eine sachgemäße Montage zu vereinfachen und ein Risiko von Leckagen zu verringern. Darüber hinaus ermöglicht ein mit dem Leitungskörper ausgestattetes Messsystem vorteilhafterweise ein einfaches Anbringen von unterschiedlichen Sensoren, sodass eine Reduktion der zu produzierenden Variantenvielfalt der Single-Use-Komponenten des Messsystems erreicht wird. Zudem ermöglicht der Leitungskörper eine Vereinfachung des Herstellungsverfahrens sowie des Sterilisationsprozesses.

Die Erfindung betrifft einen Behälter zur Aufbewahrung, Mischung und/oder Kultivierung eines Mediums, insbesondere einen Bioreaktor für ein Medium, mit einem Leitungssystem als Ableitung, Zuleitung und/oder Bypassleitung des Behälters, wobei das Leitungssystem zumindest umfasst: einen Leitungskörper, welcher einstückig ausgebildet und geeignet ist, von einem Medium durchströmt zu werden, wobei der Leitungskörper aufweist: einen ersten und einen zweiten Anschlussbereich zum Anschließen insbesondere an den Behälter und/oder das Leitungssystem; und wenigstens eine erste und eine zweite Koppeleinrichtung im Bereich zwischen den Anschlussbereichen, wobei die erste und zweite Koppeleinrichtung strukturell unterschiedlich ausgestaltet und dazu ausgebildet sind, um mit jeweils strukturell unterschiedlichen Messeinrichtungen gekoppelt zu werden.

Ein Behälter im Sinne dieser Erfindung ist ein Behältnis bzw. Container bzw. Kessel, welcher insbesondere zur Aufbewahrung, Mischung, Temperierung und/oder Kultivierung eines Mediums geeignet ist. Insbesondere kann ein solcher Behälter zur Verwendung als Einweg- oder Mehrweg-Bioreaktor geeignet sein.

Das Leitungssystem des Behälters ist insbesondere dafür geeignet, ein Medium aus dem Behälter abzuleiten bzw. abzulassen und/oder ein Medium dem Behälter zuzuführen bzw. in den Behälter zu leiten. Alternativ und/oder zusätzlich kann das Leitungssystem eine Schleifenleitung/Bypassleitung aufweisen, welche ein in dem Bioreaktor befindliches Medium umleitet, d.h. aus dem Behälter herausleitet und an anderer Stelle dem Behälter wieder zuführt. Eine solche Bypassleitung ist insbesondere vorteilhaft, da ein Medium des Behälters durch ein außerhalb des Behälters befindliches Messsystem geleitet werden kann um den Kultivierungsvorgang des Mediums zu kontrollieren und gegebenenfalls die Prozessparameter anzupassen. Das Medium des Behälters ist beispielsweise eine Flüssigkeit, ein Liquid, eine Lösung, eine Suspension, eine Dispersion, eine Emulsion, eine heterogene/homogene Stoffgemische und/oder ein Gas. Der bevorzugt im Wesentlichen geradlinig und/oder rohrförmig/rund ausgebildete Leitungskörper weist an beiden Enden einen Anschlussbereich auf, an bzw. mit welchem der Leitungskörper an weitere Komponenten des Leitungssystem angeschlossen bzw. mit diesen im Wesentlichen leckdicht verbunden werden kann. Alternativ und/oder zusätzlich kann der Leitungskörper an den Behälter angeschlossen sein bzw. mit diesem direkt verbunden sein. Insbesondere kann der Leitungskörper ohne gekoppelte Messeinrichtungen in dem Leitungssystem vorgesehen bzw. an das Leitungssystem/den Behälter angeschlossen sein.

Bevorzugt weist der Leitungskörper zumindest eine dritte Koppeleinrichtung auf. Dabei sind besonders bevorzugt die erste, zweite und dritte Koppeleinrichtung strukturell unterschiedlich ausgestaltet. Besonders bevorzugt ist jede der Koppeleinrichtungen des Leitungskörpers derart ausgestaltet, dass ausschließlich jeweils eine bestimmte Messeinrichtung mit diesen gekoppelt werden können. Besonders bevorzugt weist der Leitungskörper weiter eine vierte Koppeleinrichtung auf. Weiter bevorzugt sind die erste, zweite, dritte und vierte Koppeleinrichtung strukturell unterschiedlich ausgestaltet. Es können auch zwei oder drei der vier Koppeleinrichtungen gleichartig ausgestaltet sein. Dies ermöglicht beispielsweise ein Anbringen von zwei oder drei identischen Messeinrichtungen an den Leitungskörper, um Redundanzmessungen zu ermöglichen. Bevorzugt erfolgt eine solche redundante Messung in einem vorderen und einem hinteren Bereich des Leitungskörpers, um eine erhöhte Messgenauigkeit zu erhalten. Weiter bevorzugt kann der Leitungskörper fünf, sechs, sieben oder mehr Koppeleinrichtungen aufweisen, wobei redundante Koppeleinrichtungen vorhanden sein können.

Von Vorteil sind die Koppeleinrichtungen in Umfangsrichtung des Leitungskörpers und/oder in Strömungsrichtung eines durch den Leitungskörper strömenden Mediums voneinander beabstandet angeordnet. Besonders bevorzugt sind die Koppeleinrichtungen gleichmäßig beabstandet und nebeneinander in Strömungsrichtung eines durch den Leitungskörper fließenden Mediums angeordnet.

Die mit den Koppeleinrichtungen des Leitungskörpers koppelbaren Messeinrichtungen sind bevorzugt insbesondere dafür geeignet sind, Temperatur, Druck, Durchflussrate, Sauerstoffgehalt, pH-Wert, Leitfähigkeit, Viskosität und/oder optische Parameter eines durch den Leitungskörper strömenden Mediums zu messen. Zusätzlich und/oder alternativ können auch andere als die oben genannten Messeinrichtungen bzw. Sensoren mit den Koppeleinrichtungen gekoppelt werden, um weitere Parameter des Mediums zu erfassen. Bevorzugt weisen dabei die unterschiedlichen Messeinrichtungen unterscheidende strukturelle Merkmale auf, sodass eine Messeinrichtung nur mit einer für diese bestimmte Koppeleinrichtung gekoppelt werden kann. Besonders bevorzugt weisen die Koppeleinrichtungen unterschiedliche optische Kennzeichnungen bzw. Merkmale auf, sodass eine Zuordnung der Messeinrichtung mit einer zugehörigen Koppeleinrichtung erleichtert und so die Handhabbarkeit weiter vereinfacht und die Prozesssicherheit weiter erhöht werden kann. Hierfür eignen sich insbesondere ein Farbcode und/oder strukturelle "Schlüssel-Schloss"-Elemente.

Der Behälter, das Leitungssystem und/oder der Leitungskörper ist bzw. sind bevorzugt zur einmaligen Verwendung bestimmt (sog. "Single-Use"-Komponente). Wie beschrieben werden Single-Use-Komponenten bei erhöhten Anforderungen bezüglich Sterilität verwendet, sodass eine Kontamination des kultivierten Mediums im Wesentlichen verhindert bzw. reduziert werden kann. Ein solcher Einweg-Behälter, insbesondere ein solcher Einweg-Bioreaktor, besteht beispielsweise aus Verbundfolie umfassend Polyester (PE), Polyamid (PA), Polypropylen (PP), Ethylenvinylacetat (EVA), Ethylen-Vinylalkohol-Copolymer (EVOH) und/oder Polyvinylchlorid (PVC). Das Leitungssystem und/oder der Leitungskörper bestehen bevorzugt im Wesentlichen aus thermoplastischen Kunststoffen, wie z.B. Polyethylin (PE), Polypropylen (PP), Polyethylenterephthalat (PET), Polybutylenterephthalat (PBT), Fluorpolymere und ähnliche Polyolefine.

Weiter betrifft die Erfindung einen Leitungskörper für ein Leitungssystem zum Ableiten, Zuleiten und/oder Umleiten eines Mediums eines Behälters, wobei der Leitungskörper einstückig ausgebildet ist und aufweist: einen ersten und einen zweiten Anschlussbereich zum Anschließen des Leitungskörpers insbesondere an einen Behälter und/oder an das Leitungssystem; und wenigstens eine erste und eine zweite Koppeleinrichtung im Bereich zwischen den Anschlussbereichen, wobei die erste und zweite Koppeleinrichtung strukturell unterschiedlich ausgestaltet und dazu ausgebildet sind, um mit jeweils strukturell unterschiedlichen Messeinrichtungen gekoppelt zu werden. Der Leitungskörper kann insbesondere ein Teil eines Leitungssystems eines Behälters wie im Vorgang beschrieben sein und insbesondere die im Vorgang beschriebenen Merkmale und Eigenschaften aufweisen.

Der einstückig ausgebildete Leitungskörper ist bevorzugt im Wesentlichen geradlinig und/oder rohrförmig/rund ausgebildet. Insbesondere vorteilhaft weist der Leitungskörper im Wesentlichen keine strömungshinderlichen Einflüsse wie z.B. Querschnittsänderungen, Schikanen, Umlenkungen, Hinterschnitte und geringe Totvolumina auf. Vorzugsweise weist der Strömungskörper eine Wandung umfassend thermoplastische Kunststoffe, wie z.B. Polyethylin (PE), Polypropylen (PP), Polyethylenterephthalat (PET), Polybutylenterephthalat (PBT) und ähnliche Polyolefine, auf. Der Leitungskörper ist bevorzugt dafür geeignet, im Spritzgussverfahren aus entsprechend geeigneten Materialen hergestellt zu werden. Weiter bevorzugt ist der Strömungskörper dazu geeignet, zumindest teilweise mithilfe eines additiven Fertigungsverfahrens, wie z.B. Stereolithographie (SL), Laser-Sintern (LS/SLS) und/oder Fused Deposition Modeling (FDM), aus dafür geeigneten Materialien, wie z.B. Kunststoffe (Thermoplaste wie Polyethylen, Polypropylen, Polylactid, ABS, PETG und thermoplastische Elastomere), Kunstharze, Keramiken und Metallen, hergestellt zu werden. Weiter bevorzugt besteht der Leitungskörper aus inerten Materialien und/oder weist eine Beschichtung der Innenwandung aus inerten Materialien auf, sodass eine Beeinflussung des durch den Leitungskörper strömenden Mediums im Wesentlichen verhindert wird.

Der Leitungskörper weist bevorzugt zumindest eine dritte Koppeleinrichtung auf. Dabei sind weiter bevorzugt die erste, zweite und dritte Koppeleinrichtung des Leitungskörpers strukturell unterschiedlich ausgestaltet. Weiter bevorzugt weist der Leitungskörper vier oder mehr Koppeleinrichtungen auf.

Von Vorteil sind die mit den Koppeleinrichtungen des Leitungskörpers koppelbaren Messeinrichtungen insbesondere dafür geeignet, Temperatur, Druck, Durchflussrate, Sauerstoffgehalt, pH-Wert, Leitfähigkeit, Viskosität und/oder optische Parameter eines durch den Leitungskörper strömenden Mediums zu messen.

Der Leitungskörper ist weiter bevorzugt zur einmaligen Verwendung bestimmt bzw. ein Einweg-/"Single-Use"-Leitungskörper. Der Leitungskörper besteht bevorzugt aus Material umfassend thermoplastische Kunststoffe, wie z.B. Polyethylin (PE), Polypropylen (PP), Polyethylenterephthalat (PET), Polybutylenterephthalat (PBT) und ähnliche Polyolefine.

Gemäß einer besonders bevorzugten Ausführungsform weist der Leitungskörper unterschiedlich ausgestaltete Koppeleinrichtungen auf. Insbesondere weist zumindest ein Bereich einer Koppeleinrichtung eine Wandstärke von weniger als etwa 3 mm, bevorzugt weniger als etwa 2 mm auf. Eine solche Ausgestaltung der Koppeleinrichtung ermöglicht beispielsweise eine Reduzierung der Messstörungen bzw. Ungenauigkeiten, welche durch die Wandung des Leitungskörpers hervorgerufen werden. Weiter bevorzugt weist zumindest ein Bereich einer Koppeleinrichtung alternativ und/oder zusätzlich eine Wärmeleitfähigkeit auf, um Messungen der Temperatur des Mediums von außerhalb des Leitungskörpers zu ermöglichen. Alternativ und/oder zusätzlich weist zumindest ein Bereich einer Koppeleinrichtung eine Beschichtung auf der Außenseite des Leitungskörpers auf, besonders bevorzugt umfassend TPE (Thermoplastische Elastomere), Silikon, NBR (Acrylnitril-Butadien-Kautschuk) oder Materialien mit ähnlichen Eigenschaften. Eine solche Beschichtung ermöglicht insbesondere eine vorteilhafte mechanische Kopplung der Messeinrichtungen mit den derart ausgestalteten Koppeleinrichtungen. Alternativ und/oder zusätzlich kann ein Bereich einer Koppeleinrichtung eine optische Durchlässigkeit aufweisen, um Messungen optischer Parameter des Mediums von außerhalb des Leitungskörpers zu ermöglichen. Ein solcher Bereich ist weist bevorzugt eine Durchlässigkeit für elektromagnetische Strahlung auf, insbesondere für Strahlung mit einer Wellenlänge im Bereich zwischen etwa 120 nm und etwa 50 µm. Insbesondere ermöglicht dies Messungen betreffend UV-Absorption, Trübung, Streuung und sonstige spektroskopische Messungen des Mediums. Weiter bevorzugt kann zumindest ein Bereich einer Koppeleinrichtung alternativ und/oder zusätzlich eine Schalldurchlässigkeit aufweisen, um Messungen der Durchflussrate des Mediums von außerhalb des Leitungskörpers zu ermöglichen. Ein solcher Bereich umfasst vorzugsweise ein Material mit einem hohen Transmissionsgrad, sodass Schallwellen mit geringem bzw. im Wesentlichen ohne Intensitätsverlust durch die Koppeleinrichtung hindurch gelangen können und das Medium im Inneren des Leitungskörpers erreichen. Weiter bevorzugt weist ein Bereich einer Koppeleinrichtung partiell Materialien mit hoher Reflektivität (akustisch und/oder optisch) auf, um eine Vergrößerung der Messtrecke zu erreichen. Insbesondere kann eine reflektierende Fläche in einem Bereich des Leitungskörpers angebracht sein, welcher einem Bereich, in welchem optische bzw. akustische Strahlung in den Leitungskörper eingeleitet wird, gegenüberliegt.

Bevorzugt weist eine Koppeleinrichtung des Leitungskörpers ein oder mehrere Öffnungen in dem Leitungskörper zum Empfangen zumindest eines Teils einer Messeinrichtung bzw. zum Einführen zumindest eines Teils einer Messeinrichtung in das Innere des Leitungskörpers auf. Eine solche Öffnung ist insbesondere geeignet zum Empfangen bzw. Beinhalten bzw. Einführen von Messeinrichtungen, welche einen direkten Kontakt mit dem Medium benötigten bzw. bei welchen ein solcher vorteilhaft ist, wie beispielsweise Temperatursensoren, Durchflusssensoren und Leitfähigkeitssensoren. Alternativ dazu kann eine solche Öffnung zur Realisierung eines Auslasses zur Probenentnahme, eines Ventils und/oder einer Dosiervorrichtung dienen.

Bevorzugt weist eine Koppeleinrichtung des Leitungskörpers eine auslenkbare Membran auf, um Messungen des Drucks innerhalb des Leitungskörpers bzw. des Mediums von außerhalb des Leitungskörpers zu ermöglichen, wobei die auslenkbare Membran bevorzugt aufweist: eine erste Seite, die dem Inneren des Leitungskörpers zugewandt ist und mit dem Medium im Inneren des Leitungskörpers in Kontakt treten kann; und eine zweite Seite, die von außerhalb des Leitungskörpers zugänglich ist. Bevorzugt ist eine solche Membran in bzw. an einer Aussparung bzw. Öffnung der Wandung des Leitungskörpers im Wesentlichen leckdicht angebracht. Alternativ zu einer Membran kommen auch andere Vorrichtungen in Betracht, welche dafür geeignet sind, den im Inneren des Leitungskörpers herrschenden Druck von außerhalb des Leitungskörpers messbar zu machen, wie beispielsweise ein federbeaufschlagter Stift.

Weiter bevorzugt weist eine Koppeleinrichtung des Leitungskörpers zwei oder mehr Elektroden auf, wobei eine erste Seite der zwei oder mehr Elektroden dem Inneren des Leitungskörpers zugewandt ist und mit dem Medium im Inneren des Leitungskörpers in Kontakt treten kann, und eine zweite Seite der zwei oder mehr Elektroden von außerhalb des Leitungskörpers zugänglich ist. Besonders bevorzugt sind die Elektroden beabstandet in Strömungsrichtung des Mediums angeordnet. Eine mit den zwei oder mehr Elektroden gekoppelte Messeinrichtung ist insbesondere dazu geeignet, die elektrische Leitfähigkeit des Mediums zu messen.

Von Vorteil weist eine Koppeleinrichtung des Leitungskörpers eine Empfangseinrichtung, insbesondere einen Schacht und/oder eine Schiene, zum Empfangen zumindest eines Teils einer Messeinrichtung auf. Eine solche Empfangseinrichtung ist bevorzugt integral mit der Wandung des Leitungskörpers ausgebildet und ermöglicht ein einfaches und sachgemäßes Positionieren und/oder Anbringen einer Messeinrichtung an der Koppeleinrichtung.

Der Leitungskörper ist weiter bevorzugt für eine Bestrahlungs- und/oder Autoklav- bzw. Dampfsterilisation geeignet. Dies ermöglicht ein besonders benutzerfreundliches Integrieren des Leitungskörpers in ein Leitungssystem bzw. Anschließen des Leitungskörpers an einen Behälter, da der Leitungskörper bereits prästerilisiert bereitgestellt werden kann. Außerdem können die Messeinrichtungen erst nach Installation des Leitungskörpers bzw. des Leitungssystems mit den Koppelvorrichtungen gekoppelt werden, sodass die Sterilität im Inneren des Leitungskörpers bzw. des Leitungssystems nicht beeinflusst wird. Insbesondere erlaubt dies das Verwenden von Mehrfach-Messeinrichtungen und ermöglicht somit eine erhebliche Kosten- und Ressourceneinsparung.

Vorteilhafterweise weist der Leitungskörper Koppeleinrichtungen auf, welche in Strömungsrichtung eines durch den Leitungskörper strömenden Mediums und/oder in Umfangsrichtung des Leitungskörpers voneinander beabstandet angeordnet sind. Besonders bevorzugt sind zwei im Wesentlichen radial gegenüberliegend angeordnete Koppeleinrichtungen.

Weiter bevorzugt weist der Leitungskörper ferner eine Geometrie im Mediumströmungsbereich des Leitungskörpers auf, welche geeignet ist, die Strömungseigenschaften des Mediums zu beeinflussen. Besonders bevorzugt weist der Bereich hydrodynamische und/oder aerodynamische Strukturen auf, welche einen Strömungstyp, welcher sich im Wesentlichen in Leitungsrichtung ausbreitet, erzeugt.

Von Vorteil ist ein Leitungskörper, welcher einen Leitungshauptkörper mit den Anschlussbereichen und einem Ausschnitt, und einen Leitungsteilkörper mit zumindest zwei Koppeleinrichtungen umfasst, wobei der Ausschnitt des Leitungshauptkörpers derart ausgestaltet bzw. dazu ausgebildet ist, den Leitungseilkörper zumindest bereichsweise zu empfangen. Dabei ist ein im Wesentliche leckdichte Zusammenfügung von Leitungshauptkörper und Leitungsteilkörper vorteilhaft. Alternativ können Leitungshauptkörper und Leitungsteilkörper jeweils zumindest eine Koppeleinrichtung aufweisen.

Weiter betrifft die Erfindung ein Messsystem für einen Behälter zur Aufbewahrung, Mischung und/oder Kultivierung eines Mediums, insbesondere einen Bioreaktor, zum Messen von Parametern eines Mediums umfassend: einen Leitungskörper wie im Vorgang beschrieben und zwei oder mehr Messeinrichtungen, welche jeweils mit einer der Koppeleinrichtungen des Leitungskörpers bevorzugt lösbar gekoppelt sind und wobei zumindest zwei der Messeinrichtungen strukturell unterschiedlich ausgebildet sind. Das Messsystem ist bevorzugt ein Teil eines Leitungssystems für einen Behälter zur Aufbewahrung, Mischung und/oder Kultivierung eines Mediums, insbesondere einen Bioreaktor, bevorzugt umfassend einen wie im Vorgang beschriebenen, einstückig ausgebildeten Leitungskörper. Besonders bevorzugt weist der Leitungskörper des Messsystems vier Koppeleinrichtungen auf, wobei zumindest drei der Koppeleinrichtungen strukturell unterschiedlich ausgebildet sind.

Besonders bevorzugt umfasst das Messsystem vier unterschiedliche Koppeleinrichtungen und vier mit diesen gekoppelte Messeinrichtungen. Von Vorteil sind die jeweiligen Koppeleinrichtungen und mit diesen gekoppelten Messeinrichtungen dafür geeignet, die folgenden Parameter des durch die Leitung strömenden Mediums zu erfassen: Druck, Durchflussrate, Leitfähigkeit und Temperatur. Zusätzlich und/oder alternativ hierzu weist das Messsystem Koppeleinrichtungen und Messeinrichtungen auf, welche geeignet sind zum Messen der Viskosität und/oder optischen Parametern, wie z.B. UV-Absorption, Trübung, Streuung und spektroskopische Parameter. Beispielhafte Ausführungen von besonders geeigneten Ausgestaltungen der Koppeleinrichtungen des Leitungskörpers sind im Vorgang beschrieben.

Im Folgenden werden einzelne Ausführungsformen zur Lösung der Aufgabe anhand der Figuren beispielhaft beschrieben. Dabei weisen die einzelnen beschriebenen Ausführungsformen zum Teil Merkmale auf, die nicht zwingend erforderlich sind, um den beanspruchten Gegenstand auszuführen, die aber in bestimmten Anwendungsfällen gewünschte Eigenschaften bereitstellen. So sollen auch Ausführungsformen als unter die beschriebene technische Lehre fallend offenbart angesehen werden, die nicht alle Merkmale der im Folgenden beschriebenen Ausführungsformen aufweisen. Ferner werden, um unnötige Wiederholungen zu vermeiden, bestimmte Merkmale nur in Bezug auf einzelne der im Folgenden beschriebenen Ausführungsformen erwähnt. Es wird darauf hingewiesen, dass die einzelnen Ausführungsformen daher nicht nur für sich genommen, sondern auch in einer Zusammenschau betrachtet werden sollen. Anhand dieser Zusammenschau wird der Fachmann erkennen, dass einzelne Ausführungsformen auch durch Einbeziehung von einzelnen oder mehreren Merkmalen anderer Ausführungsformen modifiziert werden können. Es wird darauf hingewiesen, dass eine systematische Kombination der einzelnen Ausführungsformen mit einzelnen oder mehreren Merkmalen, die in Bezug auf andere Ausführungsformen beschrieben werden, wünschenswert und sinnvoll sein kann und daher in Erwägung gezogen und auch als von der Beschreibung umfasst angesehen werden soll.

### Kurze Beschreibung der Zeichnungen

- **Figur 1**: zeigt eine beispielhafte Ausführungsform eines Behälters zur Aufbewahrung, Mischung und/oder Kultivierung eines Mediums, beispielsweise eines Bioreaktors, mit einem Leitungssystem als Ableitung, Zuleitung und Bypassleitung, wobei das Leitungssystem einen einstückig ausgebildeten Leitungskörper umfasst;
- **Figur 2**: zeigt eine perspektivische Ansicht einer beispielhaften Ausführungsform eines Leitungskörpers für ein Leitungssystem mit vier Koppeleinrichtungen und einer mit einer der Koppeleinrichtungen gekoppelten Messeinrichtung;
- **Figur 3**: zeigt eine perspektivische Ansicht einer beispielhaften Ausführungsform eines Messsystems für einen Behälter zur Aufbewahrung, Mischung und/oder Kultivierung eines Mediums mit einem Leitungskörper umfassend vier Koppeleinrichtungen und vier an diese gekoppelte Messeinrichtungen;
- **Figur 4**: zeigt einen Schnitt einer beispielhaften Ausführungsform eines Leitungskörpers mit einer Koppeleinrichtung und einer gekoppelten Messeinrichtung zum Messen der Temperatur eines Mediums;
- **Figur 5**: zeigt einen Schnitt einer weiteren beispielhaften Ausführungsform eines Leitungskörpers mit einer Koppeleinrichtung und einer gekoppelten Messeinrichtung zum Messen der Leitfähigkeit eines Mediums.
- **Figur 6**: zeigt eine weitere beispielhafte Ausführungsform eines Leitungskörpers eines Messsystems mit zwei Koppeleinrichtungen, welche gegenüberliegend voneinander angeordnet sind;
- **Figur 7**: zeigt eine alternative beispielhafte Ausführungsform eines Leitungskörpers, wobei zwei Koppeleinrichtungen in Strömungsrichtung des Mediums hintereinander beabstandet angeordnet sind;
- **Figur 8**: zeigt eine beispielhafte Ausführungsform eines Leitungskörpers, welcher einen Leitungshauptkörper und einen Leitungsteilkörper mit zwei Koppeleinrichtung aufweist;
- **Figur 9**: zeigt einen Längsschnitt eines Leitungskörpers gemäß der Ausführungsform der Figur 8, wobei der Leitungskörper eine strömungsbildverändernde Geometrie im Mediumströmungsbereich aufweist;
- **Figur 10**: zeigt eine weitere beispielhafte Ausführungsform eines Leitungskörpers mit einer Koppeleinrichtung umfassend eine Beschichtung;
- **Figur 11A**: zeigt einen Leitungskörper gemäß einer Ausführungsform der Figur 10 im Längsschnitt;
- **Figur 11B**: zeigt eine alternative Ausführungsform des Leitungskörpers der Figur 10 im Längsschnitt;
- **Figur 11C**: zeigt eine weitere alternative Ausführungsform des Leitungskörpers der Figur 10 im Längsschnitt.

### Detaillierte Beschreibung der Zeichnungen

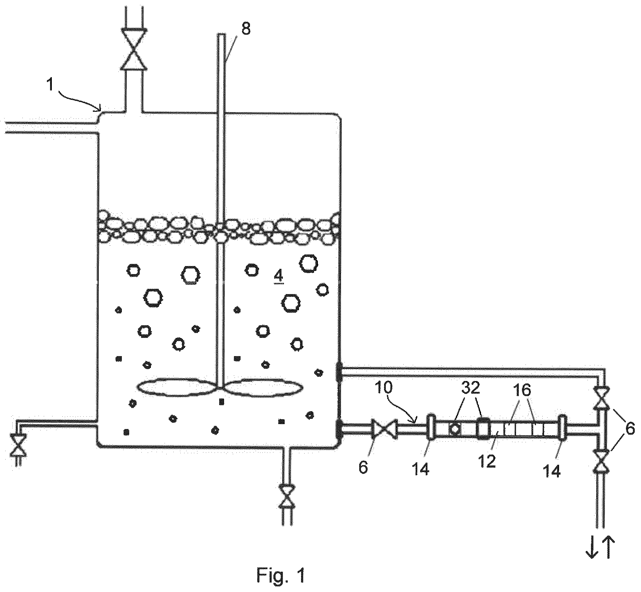

Die **Figur 1** zeigt einen Behälter 1 zur Aufbewahrung, Mischung und/oder Kultivierung eines Mediums, beispielsweise einen Bioreaktor, gemäß einer bevorzugten Ausführungsform der Erfindung. Der gezeigte Behälter 1 ist ein Einweg-Behälter bestehend beispielsweise aus Polyester (PE), Polyamid (PA), Polypropylen (PP), Ethylenvinylacetat (EVA), Ethylen-Vinylalkohol-Copolymer (EVOH) und/oder Polyvinylchlorid (PVC). Alternativ ist auch ein Mehrwegreaktor beispielsweise aus Glas und/oder Metall/Edelstahl geeignet (z.B. UniVessel^{®} Glass/SU). Alternativ kann ein solcher Behälter in einer Plattform (z.B. ambr^{®}250) bzw. in einem Gehäuse bereitgestellt werden (z.B. Flexsafe STR^{®} mit BIOSTAT STR^{®} System) oder zusammen mit einer Kontrolleinheit (z.B. UniVessel^{®} Glass/SU mit BIOSTAT^{®} A/B/Cplus, Flexsafe^{®} RM mit BIOSTAT^{®} RM) verwendet werden. Der Behälter 1 enthält ein Medium 4, wobei das Medium 4 bevorzugt ein Liquid, eine Lösung, eine Suspension, eine Dispersion, eine Emulsion und/oder ein heterogenes/homogenes Stoffgemisch ist. Bevorzugt weist der Behälter 1 eine Rührvorrichtung 8 zum Umwälzen des Mediums 4 auf, sowie ein oder mehrere Zu- und/oder Ableitungen zum Zuführen bzw. Ableiten von Medien. Der Behälter 1 gemäß der dargestellten, bevorzugten Ausführungsform weist weiter ein Leitungssystem 10 auf. Das Leitungssystem 10 kann insbesondere ein oder mehrere Ventile 6 umfasst und/oder mittels solcher mit dem Behälter 1 verbunden sein. Das Leitungssystem 10 kann als Zuleitung, Ableitung und/oder Bypassleitung ausgebildet sein. Gemäß der dargestellten bevorzugten Ausführungsform umfasst das Leitungssystem 10 mehrere Ventile, um einen Fluss eines Mediums in dem Leitungssystem 10 zu steuern, sodass ein Medium von dem Leitungssystem 10 wahlweise dem Behälter 1 zugeleitet oder aus dem Behälter 1 abgeleitet werden kann. Ebenfalls ist ein Umleiten des Mediums 4 des Behälters 1 möglich, wobei das Medium 4 an einer Stelle des Behälters 1 aus diesem herausgeleitet und an einer anderen Stelle des Behälters wieder hineingeleitet wird. Weitere mögliche Ausführungsformen einer Zu-/Ab-/Bypassleitung umfassen Filtrationsleitungen, beispielsweise mit Filtermodulen (Alternating Tangential Flow Filtration (ATF)/Tangential Flow Filtration (TFF)), um bestimmte gefilterte Bestandteile des Mediums abzuleiten und andere Bestandteile des Mediums wieder dem Behälter zuzuführen. Das Leitungssystem 10 besteht bevorzugt im Wesentlichen aus thermoplastischen Kunststoffen, wie z.B. Polyethylin (PE), Polypropylen (PP), Polyethylenterephthalat (PET), Polybutylenterephthalat (PBT) und ähnlichen Polyolefinen. Das Leitungssystem 10 kann dabei eine unterschiedliche Komponenten, insbesondere Leitungskomponenten, Ventile und/oder Anschluss- bzw. Verbindungskomponenten umfassen.

Das in Figur 1 gezeigte Leitungssystem 10 weist ferner einen einstückig ausgebildeten Leitungskörper 12 auf, der durch Medium durchströmt werden kann, das von und/oder zu dem Behälters 1 strömt bzw. fließt. Der Leitungskörper 12 ist bevorzugt im Wesentlichen rohrförmig und/oder geradlinig ausgebildet. Jedoch kann dieser auch beispielsweise gebogen und/oder oval ausgebildet sein. Der Leitungskörper 12 weist an beiden Enden Anschlussbereiche 14 auf, über bzw. mit welchen der Leitungskörper 12 an andere Komponenten des Leitungssystems 10 angeschlossen bzw. mit diesen verbunden werden kann. Bevorzugt werden Sterilkonnektoren (z.B. Lynx^{®} Konnektor, Opta^{®} Konnektor) verwendet, um den Leitungskörper 12 in das Leitungssystem 10 zu integrieren und/oder eine im Wesentlichen leckdichte und sterile Verbindung zwischen den Komponenten des Leitungssystems 10 und dem Leitungskörper 12 zu erreichen. Alternativ und/oder zusätzlich kann der Leitungskörper 12 beispielsweise an den Behälter 1 angeschlossen werden und/oder an eine externe Leitung. Ebenso kann das Anschließen des Leitungskörpers 12 alternativ und/oder zusätzlich beispielsweise mit einem Klemmverbinder (Tri-Clamp), einem Adapter, einer Kupplung (BSP), einer Schlauchtülle (Hose Barb), einem Schnellverbinder, einem Stecknippel und/oder einem Gewinde erfolgen. Bevorzugt können geeignete Dichtungen bzw. Dichtmittel verwendet werden, um eine im Wesentlichen leckdichte Verbindung zwischen den einzelnen Komponenten des Leitungssystems 10 und/oder zu dem Behälter 1 zu erhalten. Ein oder mehrere Anschlussbereiche 14 des Leitungskörpers 12 können im Wesentlichen identisch oder unterschiedlich ausgestaltet sein. Insbesondere können Art, Durchmesser, Länge und/oder Form beliebig abweichen. Ebenso kann der Durchmesser eine oder mehrerer Teile der Anschlussbereiche 14 mehr oder weniger stark von einem Durchmesser des Leitungskörpers 12 abweichen.

Die in der Figur 1 dargestellte bevorzugte Ausführungsform des Leitungskörpers 12 weist eine Vielzahl von z.B. vier Koppeleinrichtungen 16 auf, wobei an zwei der vier Koppeleinrichtungen 16 jeweils eine Messeinrichtung 32 gekoppelt sind. Dabei können Anzahl und/oder Art der an den Leitungskörper 12 gekoppelten Messeinrichtungen 32 je nach Anwendung variieren. Dabei sind die Koppeleinrichtungen 16 des Leitungskörpers 12 voneinander beabstandet (bevorzugt in im Wesentlichen regelmäßigen Abständen) in Längsrichtung des Leitungskörpers 12 bzw. entlang einer Flussrichtung eines durch den Leitungskörper 12 strömenden Mediums angeordnet. Die Koppeleinrichtungen 16 sind bevorzugt strukturell unterschiedlich voneinander ausgestaltet, sodass eine vorteilhafte Zuordnung zwischen einer Koppeleinrichtung 16 und einer entsprechend ausgestalteten Messeinrichtung 32 ermöglicht wird. Es ist somit vorteilhaft möglich, unterschiedliche Messeinrichtungen 32 an einer jeweils entsprechenden Koppeleinrichtung 16 zu montieren bzw. koppeln.

Besonders bevorzugt ist eine Koppeleinrichtung 16 derart ausgestaltet, dass sich diese zur vorteilhaften Kopplung mit unterschiedlichen Messeinrichtungen 32 eignet. Die mit bzw. an die Koppeleinrichtungen 16 des Leitungskörpers 12 gekoppelten bzw. angebrachten Messeinrichtungen 32 sind insbesondere dafür geeignet, Temperatur, Druck, Durchflussrate, Leitfähigkeit, Viskosität und/oder optische Parameter eines durch den Leitungskörper 12 strömenden Mediums zu messen. Der Leitungskörper 12 stellt somit eine universelle Komponente des Leitungssystems 10 zum Anbringen von einer Vielzahl von Messeinrichtungen 32 in einer Vielzahl von Kombinationsmöglichkeiten dar. Im Weitern werden einige beispielhafte und besonders bevorzugte Ausgestaltungen von Koppeleinrichtungen 16 und Messeinrichtungen 32 beschrieben.

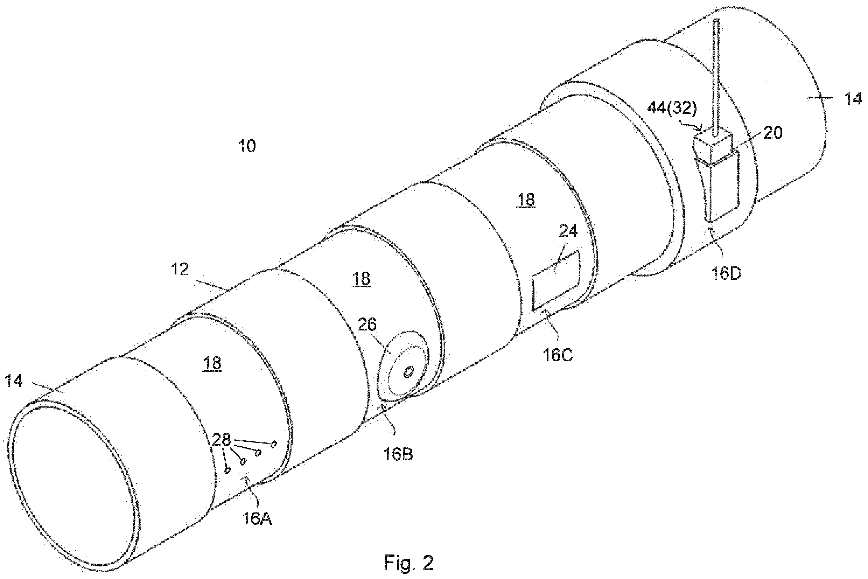

Die **Figur 2** zeigt eine beispielhafte und bevorzugte Ausführungsform eines einstückig und im Wesentlichen rohrförmig ausgebildeten Leitungskörpers 12 eines Leitungssystems 10. Der Leitungskörper 12 ist Teil eines Leitungssystems 10 bzw. als Teil eines Leitungssystems 10 vorgesehen, welches ein Medium einem Behälter 1 zuführen und/oder aus einem Behälter 1 ableiten kann. Bevorzugt ist der Leitungskörper 12 (z.B. durch Gammastrahlung und/oder Autoklavieren) sterilisierbar ausgestaltet. Der Leitungskörper 12 weist an beiden Enden einen Anschlussbereich 14 auf, durch welchen der Leitungskörper 12 an Komponenten des Leitungssystems 10 und/oder an einen Behälter angeschlossen werden kann bzw. in das Leitungssystem integriert werden kann. Der Leitungskörper 12 weist zumindest zwei (z.B. vier) Koppeleinrichtungen 16A-16D auf, wobei die Koppeleinrichtungen 16A-16D bevorzugt strukturell unterschiedlich ausgestaltet sind. In der gezeigten Ausführungsform sind die Koppeleinrichtungen 16 entlang der Längsachse des Leitungskörpers 12 mit im Wesentlichen gleichen Abständen zueinander angeordnet.

Die Koppeleinrichtung 16A weist in der gezeigten bevorzugten Ausführungsform eine Vielzahl (z.B. vier) Elektroden 28 auf, welche in die Wandung des Leitungskörpers 12 eingebracht sind. Bevorzugt erstrecken sich die einzelnen Elektroden 28 von dem Innenumfang des Leitungskörpers 12 zu dessen Außenumfang, sodass die Elektroden 28 einer Durchkontaktierung durch den Leitungskörper 12 entsprechen. Die Elektroden 28 sind von außerhalb des Leitungskörpers 12 zugänglich, sodass eine Messeinrichtung 32 mit entsprechend ausgestalteten Kontaktelementen Signale von den Elektroden 28 abgreifen bzw. Signale an die Elektroden 28 übertragen kann. Die Elektroden 28 werden bevorzugt in die Wandung des Leitungskörpers während dessen Herstellung eingebettet. Alternativ können Elektroden in Öffnungen in der Wandung des Leitungskörpers eingebracht werden. Die Elektroden können plan mit dem Innen und/oder Außenumfang des Leitungskörpers abschließen oder von der Wandung des Leitungskörpers hervorragen. Die Elektroden umfassen bevorzugt rostfreien Stahl/Edelstahl, Platin und/oder Titan oder Materialien mit ähnlichen Eigenschaften, insbesondere ähnlicher elektrischer Leitfähigkeit. Vorzugsweise sind die Materialien im Wesentlichen (chemisch) inert gegenüber den Bestandteilen des Mediums bzw. weisen im Wesentlichen keinen zellschädigenden Einfluss auf Bestandteile des Mediums auf. Die Koppeleinrichtung 16A ist insbesondere dafür geeignet, mit einem Leitfähigkeitssensor gekoppelt zu werden, um die Leitfähigkeit eines in dem Leitungskörper 12 befindlichen bzw. durch den Leitungskörper 12 strömenden Mediums zu Messen. Die Koppeleinrichtung 16A weist darüber hinaus bevorzugt zumindest bereichsweise eine Oberflächenbeschichtung 18 auf, welche eine weiche Kontaktfläche für eine vorteilhafte mechanische Kopplung einer Messeinrichtung 32, insbesondere von Befestigungsvorrichtungen zum Anbringen an die Koppeleinrichtung 16 des Leitungskörpers 12, bereitstellt. Des Weiteren weist die Koppeleinrichtung 16 in der gezeigten bevorzugten Ausführungsform einen im Vergleich zum Leitungskörper 12 dünnere Wandung auf. Die Wandung der Koppeleinrichtung 16 ist bevorzugt kleiner als etwas 2 mm. Dadurch ist die Koppeleinrichtung 16 gegenüber dem Leitungskörper 12 weiter strukturell abgesetzt bzw. unterscheidend ausgestaltet.

Eine weitere besonders bevorzugte Ausführungsform einer Koppeleinrichtung 16 zeigt die Koppeleinrichtung 16B, welche eine in den Leitungskörper 12 eingefügte auslenkbare Membran 26 aufweist. Die Membran 26 umfasst bevorzugt TPE (Thermoplastische Elastomere), Silikon, NBR (Acrylnitril-Butadien-Kautschuk) oder Materialien mit ähnlichen Eigenschaften. Die Membran 26 steht mit dem durch den Leitungskörper 12 strömenden Medium in Kontakt und wird entsprechend des in dem Leitungskörper 12 befindlichen Drucks (bevorzugt im Wesentlichen in Radialrichtung des Leitungskörpers 12) ausgelenkt bzw. verformt. Die Koppeleinrichtung 16B ist insbesondere dafür geeignet bzw. ausgestaltet, mit einem Drucksensor gekoppelt zu werden, welcher den von der Membran 26 übertragenen Druck und/oder die Auslenkung der Membran 26 messen bzw. bestimmen kann.

Die Membran 26 ist bevorzugt zumindest teilweise in eine Aussparung bzw. Öffnung des Leitungskörpers 12 eingefügt und zum Leitungskörper 12 hin abgedichtet. Die Membran 26 ist bevorzugt derart ausgestaltet, dass die maximale Auslenkung der Membran 26 begrenzt ist, um einen Defekt der Membran 26 zu verhindern, z.B. falls im Betrieb des Leitungssystems 10 keine Messeinrichtung 32 mit der Membran 26 gekoppelt ist. Die Koppeleinrichtung 16B weist ferner bevorzugt eine Oberflächenbeschichtung 18 auf, um eine vorteilhafte mechanische Kopplung mit einer zu koppelnden Messeinrichtung zu ermöglichen. Eine mit der Membran 26 gekoppelte Messeinrichtung kann bevorzugt die Auslenkung der Membran 26 bzw. den von der Membran 26 auf die Messeinrichtung 32 übertragenen Druck messen bzw. detektieren. Als Messeinrichtung 32 sind insbesondere piezoelektrische, kapazitive, induktive und/oder piezoresistive Sensoren geeignet. Alternativ zu der dargestellten Membran 26 kommen auch andere Vorrichtungen in Betracht, welche dafür geeignet sind, den im Inneren des Leitungskörpers 12 herrschenden Druck von außerhalb des Leitungskörpers 12 messbar bzw. detektierbar zu machen, beispielsweise ein federbeaufschlagter Stift, welcher im Wesentlichen radial aus dem Leitungskörper 12 heraussteht.

Die Koppeleinrichtung 16C zeigt eine weitere beispielhafte und besonders bevorzugte Ausführungsform einer Koppeleinrichtung 16. Die Koppeleinrichtung 16C weist zumindest ein optisches Fenster 24 auf, beispielsweise in der Form eines Glassubstrats bzw. einer Glasscheibe. Das optische Fenster 24 kann beispielsweise in einen Ausschnitt bzw. in eine Aussparung des Leitungskörpers 12 im Wesentlichen leckdicht eingefügt bzw. eingesetzt sein. Das gezeigte optische Fenster 24 ermöglicht beispielsweise das Einleiten von elektromagnetischer Strahlung in das Innere des Leitungskörpers 12 und/oder das Detektieren von elektromagnetischer Strahlung, welche aus dem Inneren des Leitungskörpers 12 durch das optische Fenster emittiert wird. Besonders bevorzugte Materialien für ein solches optisches Fenster 24 ist Quarz, Saphir, Borosilikat. Zusätzlich kann das optische Fenster 24 ein oder mehrere verschiedene Beschichtungen, insbesondere optische Filter, aufweisen.

Das optische Fenster 24 gemäß einer bevorzugten Ausführungsform weist eine optische Durchlässigkeit auf, die eine optische Messung des innerhalb des Leitungskörpers 12 befindlichen Mediums erlaubt. Bevorzugt kann elektromagnetische Strahlung einer spezifischen (vorgegebenen bzw. vorgebbaren) Frequenz über das optische Fenster 24 (bevorzugt im Wesentlichen ohne Intensitätsverluste) in das Innere des Leitungskörpers 12 geleitet werden, um mit dem Medium zu interagieren. Ebenfalls kann aus dem Inneren des Leitungskörpers 12 heraustretende Strahlung außerhalb der Leitung 1 detektiert werden.

Gemäß der gezeigten bevorzugten Ausführungsform ist das optische Fenster 24 im Wesentlichen transparent für Strahlung mit einer Wellenlänge im Bereich zwischen etwa 120 nm und etwa 50 µm.

Die Koppeleinrichtung 16C ist insbesondere dafür geeignet, mit einer optischen Messeinrichtung 32 gekoppelt zu werden, um Messungen von optischen Parametern des Mediums innerhalb des Leitungskörpers 12 durchzuführen. Eine solche optische Messeinrichtung 32 weist bevorzugt eine Vorrichtung zum Einleiten von elektromagnetischer Strahlung und/oder zumindest einen optischen Sensor zum Detektieren von elektromagnetischer Strahlung auf.

Alternativ zu der gezeigten Ausführungsform kann eine insbesondere für optische Messungen geeignete Koppeleinrichtung 16 ein zweites optisches Fenster 24 aufweisen, welches im Wesentlichen radial gegenüberliegend zu dem ersten optischen Fenster 24 in dem Leitungskörper 12 vorgesehen ist, sodass elektromagnetische Strahlung von einer Messeinrichtung 32 durch eines der optischen Fenster 24 eingeleitet und durch das (bevorzugt im Wesentlichen gegenüberliegende) weitere optische Fenster 24 heraustreten kann, um von einer an diesem Fenster 24 gekoppelte bzw. angeordnete Messeinrichtung 32 detektiert zu werden. Eine solche vorteilhafte Ausgestaltung ermöglicht somit ein "Durchleuchten" des Mediums im Inneren des Leitungskörpers 12, um beispielsweise einen Grad einer Absorption von Licht (z.B. zumindest einer bestimmten Wellenlänge) durch das Medium im Leitungskörper 12 zu ermitteln. Alternativ oder zusätzlich kann das Einleiten und Detektieren durch dieselbe Messeinrichtung 32 erfolgen.

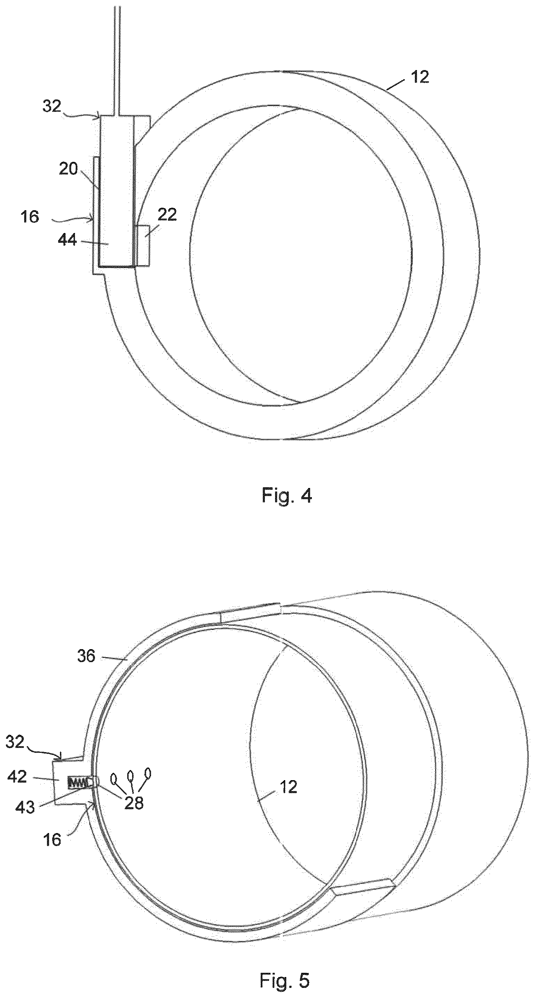

Eine weitere bevorzugte Ausführungsform einer Koppeleinrichtung 16 zeigt die Koppeleinrichtung 16D, welche eine Aufnahme- bzw. Empfangseinrichtung 20 in Form eines Schachts bzw. einer Tasche aufweist. Alternativ kann die Empfangseinrichtung 20 abweichende Merkmale aufweisen, insbesondere solche, welche ein Anbringen und/oder Positionieren einer Messeinrichtung 32 an der Koppeleinrichtung 16 des Leitungskörpers 12 ermöglichen bzw. vereinfachen, wie beispielsweise eine Schiene und/oder eine Klammer. Die dargestellte Aufnahme- bzw. Empfangseinrichtung 20 ist insbesondere dafür geeignet ist, eine Messeinrichtung 32 zumindest teilweise zu empfangen bzw. aufzunehmen. Die Messeinrichtung 32 kann lösbar oder nicht lösbar in der Empfangseinrichtung 20 angebracht bzw. aufgenommen sein. Bevorzugt ist die Empfangseinrichtung 20 der Koppeleinrichtung 16D derart ausgestaltet, dass eine in bzw. an der Empfangseinrichtung 20 positionierte Messeinrichtung 32 mit einem für die jeweilige Messung vorteilhaften Bereich der Koppeleinrichtung 16D gekoppelt ist. Beispielsweise ist ein Temperatursensor, welcher in bzw. an der Empfangseinrichtung 20 positioniert ist, mit einem Bereich der Koppeleinrichtung 16D gekoppelt, welcher eine besonders genaue Messung der Temperatur des Mediums aufgrund seiner Wärmeleitfähigkeit zulässt. Eine entsprechende besonders bevorzugte Ausführungsform ist in **Figur 4** dargestellt. Die Koppeleinrichtung 16C der gezeigten bevorzugten Ausführungsform weist darüber hinaus eine Wandung auf, welche eine größere Stärke im Vergleich zu der Wandung des Leitungskörpers 12 hat, wodurch sich diese gegenüber dem Leitungskörpers 12 weiter strukturell unterscheidet.

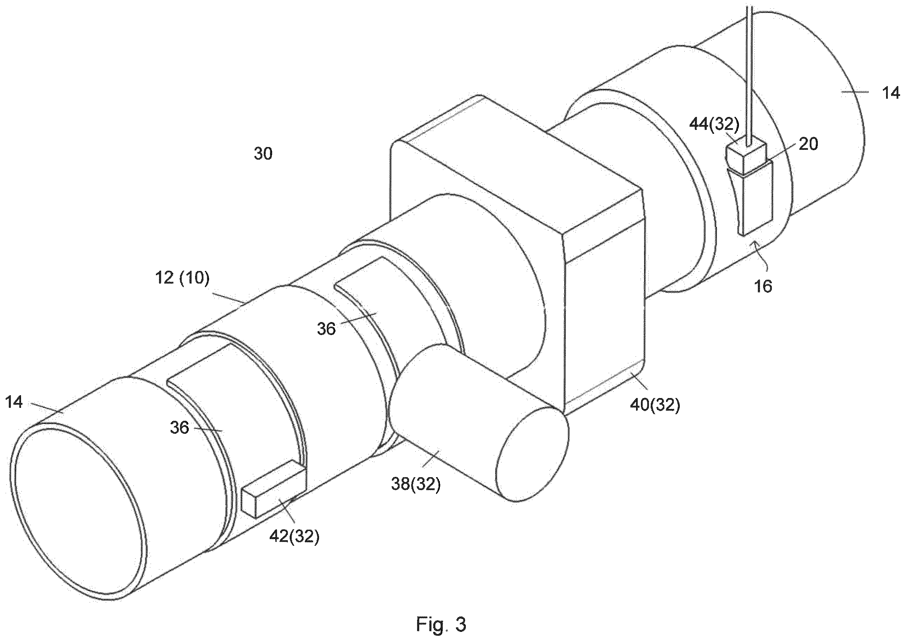

Die **Figur 3** zeigt eine beispielhafte und besonders bevorzugte Ausführungsform eines Messsystems 30 für einen Behälter 1, wie z.B. einen Bioreaktor. Bevorzugt ist das Messsystem 30 Teil eines Leitungssystems 10 für einen Behälter 1 (z.B. entsprechend Figur 1). Das Messsystem 30 umfasst weiter einen Leitungskörper 12, welcher bevorzugt im Wesentlichen rohrförmig und/oder geradlinig ausgebildet ist. Jedoch kann der Leitungskörper 12 auch abweichend davon ausgestaltet sein. In der gezeigten bevorzugten Ausführungsform des Messsystems 30 weist dieses darüber hinaus eine Vielzahl von (z.B. vier) Messeinrichtungen 38, 40, 42 und 44 auf, welche jeweils an eine Koppeleinrichtung 16 des Leitungskörpers 12 des Leitungssystems 10 montiert sind bzw. mit jeweils einer dieser Koppeleinrichtungen 16 gekoppelt sind. Die Messeinrichtungen 38, 40, 42 und 44 sind bevorzugt lösbar mit den Koppeleinrichtungen 16 gekoppelt. Weiter bevorzugt weist der Leitungskörper 12 an beiden Enden Anschlussbereiche 14 auf, um den Leitungskörper 12 in das Leitungssystem 10 eines Behälters einzusetzen bzw. zu integrieren. Dafür werden bevorzugt Sterilkonnektoren (z.B. Lynx^{®}, Opat^{®}) verwendet, um eine im Wesentlichen leckdichte Verbindung zwischen dem Leitungskörper 12 und beispielsweise Komponenten des Leitungssystems 10 bzw. einem Behälter 1 zu erreichen. Alternativ und/oder zusätzlich können beispielsweise ein Klemmverbinder (Tri-Clamp), ein Adapter, eine Kupplung, eine Schlauchtülle (Hose Barbe), ein Schnellverbinder, ein Stecknippel und/oder ein Außen- und/oder Innengewinde vorgesehen sein, um das Messsystem 30 bzw. den Leitungskörper 12 in das Leitungssystem 10 einzusetzen. Bevorzugt werden Dichtungen bzw. Dichtmittel verwendet, um eine im Wesentlichen leckdichte Verbindung zu erreichen.

Eine Messeinrichtung 32 der gezeigten Ausführungsform des Messsystems 30 umfasst einen Drucksensor 38, welcher mit einer auslenkbaren Membran 26 des Leitungskörpers 12, wie in der Figur 2 gezeigt, gekoppelt ist. Gemäß der einer bevorzugten Ausführungsform umfasst der Drucksensor 38 einen piezoelektrischen Sensor. Der Drucksensor 38 ist mit einer Befestigungsvorrichtung 36 an der Koppeleinrichtung 16 des Leitungskörpers derart (bevorzugt lösbar) angebracht, dass der piezoelektrische Sensor mit der auslenkbaren Membran 26 mechanisch gekoppelt ist, um eine Übertragung des Drucks von dem Medium im Inneren des Leitungskörpers 12 über die Membran 26 auf den Drucksensor 38 zu ermöglichen. Alternative Messeinrichtungen zur Messung des Drucks des Mediums umfassen beispielsweise kapazitive, induktive und/oder piezoresistive Sensoren.

Bei der bevorzugten Ausführungsform in Figur 3 umfasst einer Messeinrichtung 32 einen Durchflusssensor 40 zum Messen der Durchflussrate des Mediums durch den Leitungskörper 12. Die gezeigte beispielhafte Ausführungsform des Durchflusssensors 40 umfasst ein Gehäuse, welches den Leitungskörper 12 vollständig umschließt. Ebenfalls können Gehäuse von Messeinrichtung 32 den Leitungskörper 12 lediglich teilweise umschließen. Bevorzugt ist der Durchflusssensor 40 dazu ausgebildet, an eine Koppeleinrichtung 16 des Leitungskörpers beispielsweise mittels eines "Clamp-On"-Mechanismus' angebracht zu werden bzw. mit dieser gekoppelt zu werden. Zumindest ein Bereich der Wandung des Leitungskörpers 12 weist im Bereich der Koppeleinrichtung 16 für einen Durchflusssensor 40 bevorzugt eine geringe Stärke auf, bevorzugt geringer als etwa 2 mm, sodass für die Messung verwendeter Ultraschall im Wesentlichen ungehindert zu dem Medium in dem Leitungskörper 12 gelangen kann. Das Gehäuse des Durchflusssensors 40 kann (wie in der Figur 3 gezeigt) auch andere zur Messung von Parametern des durch den Leitungskörper 12 fließenden bzw. strömenden Mediums geeignete Sensoren beinhalten und/oder zudem auf unterschiedliche Arten ausgestaltet sein, beispielsweise eine runde Form aufweisen.

Eine weitere Messeinrichtung 32 des gezeigten Messsystems 30 umfasst bevorzugt einen Leitfähigkeitssensor 42, welcher vorzugsweise mit einer Koppeleinrichtung 16 des Leitungskörpers mit (bevorzugt vier) in den Leitungskörper 12 integrierten Elektroden 28 gekoppelt ist und so eine Messung der Leitfähigkeit des Mediums innerhalb des Leitungskörpers 12 erlaubt. Der abgebildete Leitfähigkeitssensor 42 umfasst bevorzugt eine Befestigungsvorrichtung 36 zur lösbaren Kopplung mit der Koppeleinrichtung 16 des Leitungskörpers 12. Der Leitfähigkeitssensor 42 weist Kontaktstellen auf, welche mit den Elektroden 28 der Koppeleinrichtung 16 in Kontakt stehen bzw. kommen. So kann ein elektrisches Signal in das Innere des Leitungskörpers 12 geleitet bzw. ein oder mehrere Signale aus dem Inneren des Leitungskörpers 12 erfasst werden. Eine besonders bevorzugte Ausführungsform einer Empfangseinrichtung 16 und gekoppelten Messeinrichtung 42 ist in **Figur 5** dargestellt.

In dem in Figur 3 gezeigten Ausführungsbeispiel weisen die Messeinrichtungen 38 und 42 jeweils eine Befestigungsvorrichtung 36 auf, um mit den Koppeleinrichtungen 16 des Leitungskörpers 12 gekoppelt zu werden, wobei das Koppeln vorzugsweise lösbar erfolgt. Bevorzugt umfassen die Befestigungsvorrichtungen 36 ein- oder mehrteilige Klammern, welche den Leitungskörper 12 an einer Koppeleinrichtung 16 bzw. in deren Umgebung zumindest teilweise umschließen. Alternativ und/oder zusätzlich können geeignete Befestigungsvorrichtungen 36 zum Anbringen bzw. Befestigen bzw. Koppeln der Messeinrichtungen 32 beispielsweise Kabelbinder und/oder einen Flansch umfassen. Ebenfalls können Befestigungsvorrichtungen 36 für Messeinrichtungen 32 zusätzlich und/oder alternativ magnetische und/oder andere Haftmittel aufweisen. Die genannten Befestigungsvorrichtungen 36 umfassen lediglich eine exemplarische Auswahl von geeigneten Befestigungsvorrichtungen 36.

Weiter weist die in der Figur 3 gezeigte bevorzugte Ausführungsform des Messsystems 30 eine Koppeleinrichtung 16 umfassend eine Empfangseinrichtung 20 auf, welche eine Messeinrichtung 32 zumindest teilweise beherbergt bzw. aufnimmt. Eine solche Empfangseinrichtung 20 ist bevorzugt integral mit der Wandung des Leitungskörpers 12 ausgebildet. Optional ist die Empfangseinrichtung 12 derart angebracht, dass eine von ihr empfangene Messeinrichtung 32 bzw. ein von der Messeinrichtung 32 umfasster Sensor 44 mit einem im Vorgang beschriebenen Bereich einer Koppeleinrichtung 16 mit vorteilhaften Eigenschaften gekoppelt werden kann. In der gezeigten bevorzugten Ausführungsform umfasst die Messeinrichtung 32 einen Temperatursensor 44 (z.B. eine PT-100 bzw. NTC/PTC Sonde). Der Temperatursensor 44 ist aufgrund der Positionierung bzw. Lagerung in der Empfangseinrichtung 20 besonders vorteilhaft mit der Koppeleinrichtung 16 zur Temperaturmessung des Mediums in dem Leitungskörper 12 gekoppelt, da insbesondere ein Lösen und/oder ein falsches Anbringen der Messeinrichtung 32 verhindert werden kann. Außerdem ermöglicht eine solche vorteilhafte Kopplung insbesondere einen verbesserten mechanischen bzw. elektrischen Kontakt zwischen Messeinrichtungen 32 und dem Leitungskörper 12 bzw. der Koppeleinrichtungen 16, sodass eine verbesserte Übertragung von beispielsweise Wärme und/oder Schallwellen und/oder elektrischen Signalen erreicht werden kann. Zusätzlich fungiert die Empfangseinrichtung 20 der Koppeleinrichtung 16 ferner bevorzugt als thermische Isolierung, sodass die Messgenauigkeit einer Temperaturmessung an der Koppeleinrichtung 16 vorteilhaft erhöht werden kann.

Neben den im Vorgang beschriebenen bevorzugten Ausführungsformen von Koppeleinrichtungen 16 und Messeinrichtungen 32 können diese auch andere Ausgestaltungen aufweisen. Eine alternative bevorzugte Koppeleinrichtung 16 weist beispielsweise ein oder mehrere Öffnungen in dem Leitungskörper 12 zum Empfangen zumindest eines Teils einer Messeinrichtung 32 bzw. zum Einführen zumindest eines Teils einer Messeinrichtung 32 in das Innere des Leitungskörpers 12 auf. Die Öffnungen sind vorteilhafterweise im Wesentlichen leckdicht verschließbar und/oder weisen eine Dichtung auf, sodass eine in der Öffnung positionierte Messeinrichtung 32 die Öffnung im Wesentlichen leckdicht verschließt. Bevorzugt können die Öffnungen beispielsweise mit einem Stopfen verschlossen werden, falls im Betrieb des Messsystems 30 keine Messeinrichtung 32 an bzw. in den Öffnungen angebracht bzw. mit diesen gekoppelt ist.

Eine weitere alternative Ausführungsform einer Koppeleinrichtung 16 ist eine Koppeleinrichtung 16, welche zwei gegenüberliegende Öffnungen aufweist. Eine solche Koppeleinrichtung 16 eignet sich insbesondere für eine Messeinrichtung 32 zur Viskositätsmessung, wobei ein Akustikwellenresonator an bzw. in der einen Öffnung und ein Akustikwellensensor an bzw. in der gegenüberliegenden Öffnung angebracht sind, um Akustikwellen in bzw. durch das Medium zu leiten bzw. zumindest einen Teil dieser Akustikwellen zu detektieren. Vorteilhafterweise weist eine Koppeleinrichtung 16, insbesondere eine zur Kopplung mit einem Akustikwellenresonater und/oder Akustikwellensensor, eine verstärkte Wandung auf, um eine ausreichende Beständigkeit gegenüber der erhöhten mechanischen Beanspruchung zu gewährleisten. Eine solche Verstärkung kann insbesondere eine Erhöhung der Wandstärke und/oder das Anbringen bzw. Einbetten verstärkender Strukturen umfassen.

Für das Messsystem 30 geeignete Messeinrichtungen 32 können unterschiedliche Ausgestaltungen insbesondere bezüglich des Empfangens und/oder Sendens von Signalen aufweisen. Die Messeinrichtungen 32 können daher unterschiedliche Mittel aufweisen, um die Messeinrichtungen 32 bzw. die von diesen umfasste Sensoren beispielsweise mit einer Steuereinheit bzw. Auswerteinheit zu verbinden und Signale, insbesondere Messsignale, zu übertragen. Dabei können sowohl mechanische Kontakte und/oder Steckverbindungen bereitgestellt werden, als auch eine kabellose Übertragung der Signale erfolgen. Ebenfalls kann eine gegebenenfalls benötigte bzw. vorteilhafte Energieversorgung mittels Batterie/Akku und/oder Kabelverbindung bereitgestellt werden.

Die **Figur 4** zeigt eine beispielhafte und besonders bevorzugte Ausführungsform einer Koppeleinrichtung 16 für eine Messeinrichtung 32 umfassend einen Temperatursensor 44 (z.B. PT100 oder NTC/PTC). Die Figur 4 zeigt ein Teilsegment eines Leitungskörpers 12. Die gezeigte Koppeleinrichtung 16 weist eine Empfangseinrichtung 20 auf, welche bevorzugt integral mit der Wandung des Leitungskörpers 12 ausgebildet ist. Alternativ und/oder zusätzlich kann eine solche Empfangseinrichtung 20 eine von dem Leitungskörper 12 separate und an diesem (bevorzugt trennbar) angeordnete Komponente sein. In der gezeigten bevorzugten Ausführungsform ist die Empfangseinrichtung 20 derart angeordnet, dass eine von ihr empfangene Messeinrichtung 32 bzw. der Temperatursensor 44 mit einem im Vorgang beschriebenen Bereich mit vorteilhaften Eigenschaften gekoppelt werden kann. Wie gezeigt weist die Empfangseinrichtung 20 eine Führung bzw. einen Schacht für die Messeinrichtung 32 bzw. den Temperatursensor 44 auf, um den Temperatursensor 44 mit einem bestimmten Bereich der Koppeleinrichtung 16 zu koppeln. Weiter bevorzugt weist eine Empfangseinrichtung 20 eine thermische Isolierung auf, um äußere Einflüsse auf einen empfangenen Temperatursensor 44 zu verringern.

Die besonders bevorzugte Ausführungsform der Koppeleinrichtung 16 weist einen für eine Temperaturmessung geeigneten Bereich auf. Bevorzugt weist ein solcher Bereich eine Wärmeleitfähigkeit von größer etwa 100 W/mK, weiter bevorzugt größer etwa 200 W/mK, weiter bevorzugt größer etwa 300 W/mK, auf und/oder stellt eine Wärmeleitbrücke dar. Eine solche Wärmeleitbrücke kann beispielsweise ein Plättchen umfassen, welches in einen Ausschnitt des Leitungskörpers 12 im Wesentlichen leckdicht eingefügt bzw. eingelassen ist, sodass das Plättchen 22 auf einer Seite mit dem Medium in Kontakt treten kann und gleichzeitig von außerhalb des Leitungskörpers 12 im Wesentlichen zugänglich ist. Eine solche Wärmeleitbrücke umfasst bevorzugt ein Material mit einer hohen Wärmeleitfähigkeit wie z.B. Aluminium, Gold, Kupfer und/oder Silber. Weiter bevorzugt weist eine solche Wärmeleitbrücke eine Wandstärke von weniger als etwa 3 mm, weiter bevorzugt weniger als etwa 2 mm, auf. Eine Abdichtung der in die Wandung des Leitungskörpers 12 eingesetzten bzw. eingelassenen Wärmeleitbrücke kann beispielsweise durch geeignete Plastomere und/oder Elastomere erfolgen. Alternativ und/oder zusätzlich kann die Wärmeleitbrücke eine in die Wandung des Leitungskörpers 12 integrierte Struktur, beispielsweise eine Gitterstruktur, aufweisen. Eine Gitterstruktur kann beispielsweise im Herstellungsprozess des Leitungskörpers 12, beispielsweise einem Spritzgussverfahren, in die Wandung des Leitungskörpers 12 eingebettet bzw. in bzw. an dieser vorgesehen werden und gegebenenfalls durch Spanen/Zerspanen freigelegt werden, sodass die Gitterstruktur mit dem Medium und/oder einer Messeinrichtung, insbesondere einem Temperatursensor 44, in Kontakt treten kann.

In der dargestellten besonders bevorzugten Ausführungsform umfasst die Koppeleinrichtung 16 ein in die Wandung des Leitungskörpers 12 eingelassenes leitfähiges Element (bevorzugt Metallplättchen) 22, welches bevorzugt im Wesentlichen plan mit der inneren Fläche des Leitungskörpers 12 abschließt und/oder mit einem in dem Leitungskörper 12 befindlichen Medium in Kontakt treten kann. Das Metallplättchen 22 weist bevorzugt eine Stärke von weniger als etwa 2 mm auf. Des Weiteren umfasst die Koppeleinrichtung 16 eine Empfangseinrichtung 20 zur Aufnahme der Messeinrichtung 32. Dabei ist die Messeinrichtung 32 bevorzugt derart mit der Koppeleinrichtung 16 gekoppelt, dass der Temperatursensor 44 mit dem Metallplättchen 22 in Kontakt steht, um eine vorteilhafte Temperaturübertragung von dem Medium über das Metallplättchen 22 zu dem Temperatursensor 44 zu ermöglichen. Alternativ und/oder zusätzlich zu dem abgebildeten Metallplättchen 22 können andere Elemente mit unterschiedlicher Form und aus unterschiedlichen Materialien bestehend eine vorteilhafte Wärmekopplung zwischen dem Temperatursensor 44 und dem in dem Leitungskörper 12 strömenden bzw. fließenden Mediums bereitzustellen. Insbesondere sind hierfür Materialien geeignet, welche eine gegenüber dem Leitungskörper 12 erhöhte Wärmeleitfähigkeit aufweisen. Alternativ und/oder zusätzlich kann die Stärke der Wandung des Leitungskörpers 12 verringert werden, um eine vorteilhafte Wärmekopplung zwischen dem Temperatursensor 44 und dem Medium zu erreichen.

Die **Figur 5** zeigt ein Teilsegment einer Koppeleinrichtung 16 des Leitungskörpers 12 und einer Messeinrichtung 32 gemäß einer exemplarischen bevorzugten Ausführungsform. Die Koppeleinrichtung 16 umfasst eine Wandung des Leitungskörpers 2 mit einer reduzierten Wandstärke von bevorzugt etwa 2 mm. Außerdem weist die Koppeleinrichtung 16 eine Vielzahl (von bevorzugt vier) in die Wandung des Leitungskörpers 12 eingelassene Elektroden 28 auf, welche von innerhalb und außerhalb des Leitungskörpers 12 kontaktierbar sind. Die an die Koppeleinrichtung 16 gekoppelte Messeinrichtung 32 umfasst einen Leitfähigkeitssensor 42 bzw. zu einem solchen gehörige Mittel zum Kontaktieren der Elektroden 28, beispielsweise federbeaufschlagte Kontakte 43. Alternativ und/oder zusätzlich können die Elektroden 28 in radialer Richtung nach außen hervorstehende Strukturen aufweisen, welche bevorzugt mit einer Messeinrichtung 32 mit geeigneten Empfangs- bzw. Kontaktelementen gekoppelt werden kann. In der dargestellten Ausführungsform ist die Messeinrichtung 32 mithilfe einer Klammer 36 mit der Koppeleinrichtung 16 des Leitungskörpers 12 gekoppelt. Bevorzugt weist die Klammer 26 strukturelle Merkmale auf, welche ein nicht sachgemäßes Anbringen der Messeinrichtung an der Koppeleinrichtung 16 bzw. des Leitungskörpers 12, insbesondere ein Anbringen, wobei die Elektroden 18 nicht mit Mittel zum Kontaktieren der Messeinrichtung 32 in Kontakt sind, verhindern. Besonders bevorzugt ist die Klammer 36 und eine zugehörige Koppeleinrichtung 16 derart ausgestaltet, dass ein korrektes Positionieren und/oder Anbringen der Klammer 36 bzw. der Messeinrichtung 32 an der Koppeleinrichtung 16 durch einfache Sichtprüfung verifiziert werden kann.

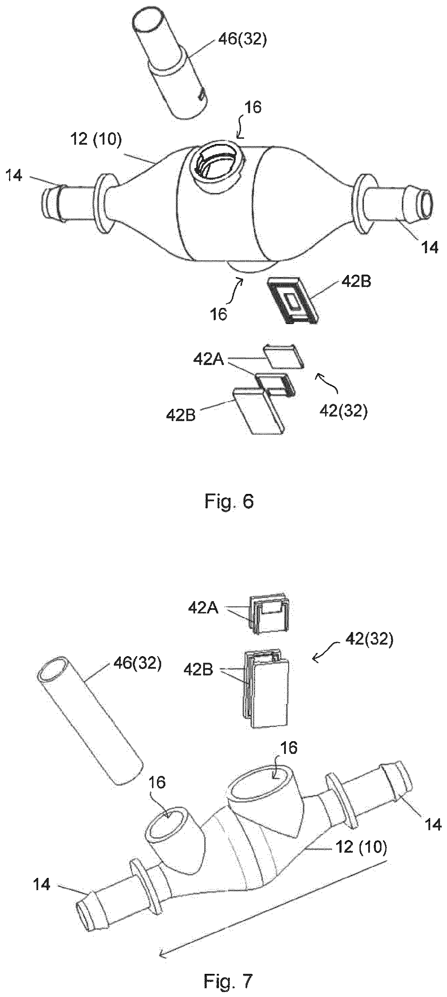

**Figur 6** zeigt eine weitere beispielhafte, besonders bevorzugte Ausführungsform eines Messsystems 10 der vorliegenden Erfindung mit zwei koppelbaren Messeinrichtungen 32. In dieser Ausführung weist der Leitungskörper 12 eine Vielzahl (z.B. zwei) Koppeleinrichtungen 16 auf, welche ihrerseits jeweils eine Öffnung zum Empfangen zumindest eines Teils einer Messeinrichtung 32 bzw. zum Einführen zumindest eines Teils einer Messeinrichtung 32 in das Innere des Leitungskörpers 12 aufweisen. Die Öffnungen der Koppeleinrichtungen 16 sind vorteilhafterweise im Wesentlichen leckdicht verschließbar und/oder weisen eine Dichtung auf, sodass eine in der Öffnung positionierte Messeinrichtung 32 die Öffnung im Wesentlichen leckdicht verschließt. Bevorzugt können die Öffnungen beispielsweise mit einem Stopfen oder einem Deckel verschlossen werden, falls im Betrieb des Messsystems 30 keine Messeinrichtung 32 an bzw. in den Öffnungen der Koppeleinrichtungen 16 angebracht bzw. mit diesen gekoppelt ist.

In der gezeigten, besonders bevorzugten Ausführungsform sind zwei Koppeleinrichtungen 16 gegenüberliegend, d.h. an im Wesentlichen radial gegenüberliegenden Bereichen des Leitungskörpers 12, angeordnet. Alternativ können zwei oder mehr Koppeleinrichtungen 16 gleichmäßig oder beliebig entlang des Umfangs des Leitungskörpers 12 angeordnet sein. Die Positionen der Koppeleinrichtungen 16 bzw. Teile davon können an im Wesentlichen identisch beabstandeten Positionen zu den Anschlussbereichen 14 angeordnet sein. Alternativ hierzu können die Positionen entlang der Länge des Leitungskörpers 12 mehr oder weniger stark variieren. Insbesondere können die Koppeleinrichtungen 16 beliebig versetzt angeordnet sein, um beispielsweise verschiedenen Anforderungen wie Messpositionen und/oder Zugänglichkeit zu genügen.

Die spezifische, vorteilhafte Anordnung der Koppeleinrichtungen 16 und der Messeinrichtungen 32 ermöglicht insbesondere eine Verhinderung, zumindest aber eine Reduzierung, einer gegenseitigen Beeinflussung der unterschiedlichen Sensoren. Darüber hinaus weist die abgebildete Ausführungsform vorteilhafterweise keine vorgegebene Ausrichtung des Leitungskörpers 12 in dem Leitungssystem 10 bezüglich der Strömungsrichtung des Mediums auf. So ist eine vereinfachte Handhabung und Montage des Messsystems 30 gewährleistet.

Das in Figur 6 gezeigte Messsystem 30 weist bevorzugt zwei Messeinrichtungen 32 auf, welche dafür geeignet sind, mit den Öffnungen der Koppeleinrichtung 16 gekoppelt bzw. zumindest teilweise von den Koppeleinrichtungen 16 empfangen zu werden. Die erste Messeinrichtung 32 umfasst beispielsweise einen pH-Sensor 46. Die zweite Messeinrichtung 32 umfasst beispielsweise einen Leitfähigkeitssensor 42 (in Explosionsdarstellung abgebildet). Vorteilhafterweise sind die Koppeleinrichtungen 16 bzw. deren Öffnungen sowie die Gehäuse der Messeinrichtungen 32 derart ausgestaltet, dass eine korrekte und stabile Positionierung bzw. Fixierung der Messeinrichtungen 32 bzw. Sensoren am bzw. im Leitungskörper 12 gewährleistet ist. Dies begünstigt sowohl die offensichtliche Kompatibilität bzw. Nicht-Kompatibilität von Messeinrichtungen 32 und Koppeleinrichtungen 12, eine im Wesentlichen leckdichte Anbringung bzw. Kopplung der Messeinrichtungen 32, als auch eine Verhinderung, zumindest aber eine Reduzierung, von störenden Einflüssen auf die Messergebnisse.

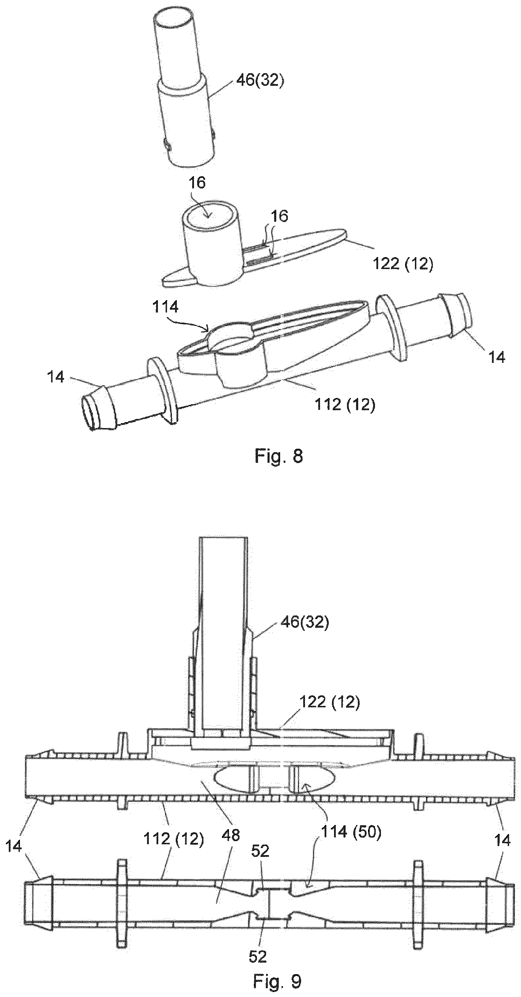

Des Weiteren ist für diese und andere Ausführungsformen der Erfindung ein reproduzierbares Strömungsbild des Mediums im jeweiligen Messbereich der Sensoren vorteilhaft. Dies kann unter anderem durch eine entsprechende Ausgestaltung des Strömungskanals innerhalb des Leitungskörpers 12 sichergestellt bzw. begünstigt werden. Eine beispielhafte und besonders bevorzugte Ausgestaltung des Strömungskanals in einem Messbereich ist in Figur 9 gezeigt. Eine solche, ähnliche oder im Wesentlichen gleichwirkende Ausgestaltung ist grundsätzlich in jedem der gezeigten sowie nicht gezeigten Ausführungsformen der Erfindung integrierbar.

Eine Koppeleinrichtung 16 des Leitungskörpers 12 in Figur 6 ist dazu ausgebildet, einen Leitfähigkeitssensor 42 zu empfangen. Der gezeigte, beispielhafte Leitfähigkeitssensor 42 umfasst jeweils eine oder mehrere (z.B. zwei) Frontplatten und Rückplatten, wobei jeweils eine Frontplatte 42A und eine Rückplatte 42B ein Plattenpaar bilden. Die Plattenpaare des abgebildeten Leitfähigkeitssensors 42 können durch die Öffnung der Koppeleinrichtung 42 in das Innere des Leitungskörpers 12 eingebracht und in dem Mediumströmungsbereich 48 positioniert werden, um die Leitfähigkeit des vorbeiströmenden Mediums zu messen. Alternativ können anders ausgestaltete Leitfähigkeitssensoren 42 und/oder Sensoren zur Messung weiterer Parameter montiert werden, insbesondere ein oder mehrere der vorgenannten Drucksensoren 38, Durchflusssensoren 40 und/oder Temperatursensoren 44. Ebenfalls können ein oder mehrere der Koppeleinrichtungen 16 eine Beschichtung 18, eine Empfangseinrichtung 20, ein Metallplättchen 22, ein Fenster 28, eine Membran 26 und/oder eine Elektrode 28 aufweisen.

In **Figur 7** ist eine weitere alternative Ausführungsform eines Leitungskörpers 12 dargestellt. Anders als der Leitungskörper 12 der Figur 6 weist der vorliegende Leitungskörper 12 eine Vielzahl (z.B. zwei) Koppeleinrichtungen 16 auf, welche in Flussrichtung des Mediums beabstandet angeordnet sind. Bevorzugt kann bei dieser Ausführungsform eine vorgegebene Montageausrichtung bestehen, sodass ein bestimmter Anschlussbereich 14 als Zulauf und der andere Anschlussbereich 14 als Ablauf festgelegt sind. Dies ist insbesondere vorteilhaft, wenn an die Koppeleinrichtungen 16 jeweils ein pH-Sensor 46 und ein Leitfähigkeitssensor 42 gekoppelt sind. Bei dieser Ausgestaltung ist vorteilhafterweise der Leitfähigkeitssensor 42 näher am Zulauf-Anschlussbereich 14 angeordnet als der pH-Sensor 46. Bevorzugt ist der pH-Sensor 46 beabstandet in Flussrichtung des Mediums am Leitungskörper 12 angeordnet. Auf diese Weise können die einzelnen Messungen der Sensoren störungsfrei, wiederholbar, kontinuierlich und genau durchgeführt werden. Insbesondere wird dadurch eine mögliche Beeinflussung der Leitfähigkeitsmessung insbesondere durch austretende Ionen aus einer Referenzelektrode der pH Messkette bzw. des pH-Sensors 46 vermieden.

Insbesondere bei einer flussrichtungsabhängigen Montagevorgabe können die Anschlussbereiche 14 unterschiedliche ausgestaltet sein, sodass ein falscher Einbau in das Leitungssystem verhindert, zumindest aber erschwert, wird.

**Figur 8** zeigt eine weitere bevorzugte Ausführungsform der Erfindung, bei welcher der Leitungskörper 12 einen Leitungshauptkörper 112 und einen Leitungsteilkörper 122 aufweist. Der Leitungshauptkörper 112 weist dabei einen Ausschnitt 114 auf, welcher derart ausgestaltet ist, dass der Leitungskörper 122 zumindest teilweise an bzw. in dem Ausschnitt 114 angeordnet werden kann. Der Leitungsteilkörper 122 weist ein oder mehrere (z.B. zwei) Koppeleinrichtungen 16 auf. Der Leitungshauptkörper 112 kann ebenfalls ein oder mehrere weitere Koppeleinrichtungen 16 aufweisen. Bei dieser vorteilhaften Ausgestaltung besteht die Möglichkeit, den Leitungsteilkörper 122 gegen einen anderen Leitungskörper 122 auszutauschen. So kann auf einfache Weise ein in ein Leitungssystem 10 integrierter Leitungskörper 12 für andere Messeinrichtungen 32 bzw. Sensoren kompatibel werden. Beispielsweise kann ein Leitungsteilkörper 122 mit Koppeleinrichtungen 16 für einen bestimmten pH-Sensor 46 und einen bestimmten Leitfähigkeitssensor 42 durch einen anderen Leitungsteilkörper 122 mit anderen Koppeleinrichtungen 16, z.B. für einen Drucksensor 38 und/oder einen Durchflusssensor 40 ausgetauscht werden, ohne den Leitungskörper 12 aus dem Leitungssystem 10 ausbauen zu müssen. Selbiges gilt für Messeinrichtungen 32, welche dieselben Parameter messen, jedoch eine andere Bauform aufweisen.

Ebenso kann ein Leitungsteilkörper 122 ohne Koppeleinrichtungen 16 als Platzhalter dienen, wobei der Leitungsteilkörper 122 ohne Koppeleinrichtungen 16 bei Bedarf gegen einen Leitungsteilkörper 122 mit einem oder mehreren Koppeleinrichtungen 16 ausgetauscht wird, um Messungen durchführen zu können. Ein Leitungskörper 12 gemäß diesem Ausführungsbeispiel weist eine gesteigerte Versatilität auf, da jeweils benötigte Koppeleinrichtungen 16 ausgetauscht und/oder nachgerüstet bzw. ergänzt werden können. Die Anordnung der einzelnen Koppeleinrichtungen 16 können auch abweichend, beispielsweise wie in Figur 6 gezeigt, d.h. mit in etwa gleichem Abstand zu einem Anschlussbereich 14, angeordnet werden.

Vorteilhafterweise sind Ausschnitt 114 und Leitungsteilkörper 122 derart ausgestaltet, dass eine im Wesentlichen leckdichte Anbringung des Leitungsteilkörpers 122 zumindest bereichsweise in bzw. an dem Ausschnitt 144 ermöglicht wird. Ebenfalls ist eine die Strömung im Inneren des Leitungskörpers 12 nicht negativ beeinflussende Anordnung bevorzugt. Außerdem kann eine nichtsymmetrische Ausgestaltung des Ausschnitts 114 und des Leitungsteilkörpers 122 vorteilhaft sein, da so eine falsche Montage bezüglich der Strömungsrichtung des Mediums verhindert werden kann.

**Figur 9** zeigt zwei Schnittansichten eines Ausführungsbeispiels der Erfindung, bei welchem der Leitungskörper 12 einen Leitungshauptkörper 112 und einen Leitungsteilkörper 122, wie zu Figur 8 beschrieben, umfasst. Darüber hinaus umfasst der Leitungskörper 12, bevorzugt der Leitungshauptkörper 112, in zumindest einem Messbereich des Mediumstroms eine strömungsbildverändernde Geometrie 50. Die Geometrie 50 weist eine strukturelle Ausgestaltung auf, welche dem durch den Messbereich fließenden Mediumstrom bestimmte Merkmale verleiht. Bevorzugt ist dabei eine hydrodynamische und/oder aerodynamische Struktur, welche Turbulenzen und/oder Rückfluss und/oder Toträume verhindert, zumindest aber deutlich reduziert. In Figur 9 ist eine beispielhafte, besonders bevorzugte Ausgestaltung einer strömungsbildverändernden Geometrie 50 gezeigt. Weitere gewünschte bzw. vorteilhafte Strömungseigenschaften sind beispielsweise Strömungsgeschwindigkeit und Flussrichtung (z.B. abweichend zur Hauptflussrichtung im Leitungskörper), welche durch entsprechende Ausgestaltungen erreicht werden.

Im Ausführungsbeispiel der Figur 9 ist die strömungsbildverändernde Geometrie 50 derart angeordnet, dass der von ihr verursachte vorteilhafte Mediumfluss in einem Messbereich erzeugt wird, in welchem beispielsweise ein wie in den Figuren 6 und 7 gezeigter Leitfähigkeitssensor 42 angebracht werden kann. Die hydrodynamischen und/oder aerodynamischen Strukturen der gezeigten strömungsbildverändernden Geometrie 50 weisen vorteilhafterweise eine im Wesentlichen symmetrische Form auf. Bevorzugt umfasst die strömungsbildverändernde Geometrie 50 eine oder mehrere Aussparungen 52, in welche ein oder mehrere Teile einer Messeinrichtung 32 bzw. eines Sensors bevorzugt im Wesentlichen passgenau eingefügt werden kann. Auf diese Weise wird die laminare Strömung im Messbereich so wenig wie möglich negativ beeinflusst. Die beschriebenen Strukturen der strömungsbildverändernden Geometrie 50 sind exemplarisch für den in den Figuren 6 und 7 gezeigten Leitfähigkeitssensor 42. Die Rückplatten 42B sitzen passgenau in den Aussparungen 52, sodass eine im Wesentlichen glatte Strömungskanalwandung im Messbereich vorhanden ist. Bei abweichenden Ausgestaltungen der Sensoren bzw. der Messeinrichtungen 32 sind entsprechend abweichende Strukturen der strömungsbildverändernden Geometrie 50 vorteilhaft.

Ausgestaltungen und/oder Anordnungen einer strömungsbildverändernden Geometrie 50 können je nach Anforderungen an das Messsystem 30 variiert werden. Ebenso kann der Strömungskörper 12 mehrere strömungsbildverändernde Geometrien 50 aufweisen, insbesondere auch in Verbindung mit anderen Koppeleinrichtungen 16, sodass auch andere Messeinrichtungen 32 bzw. Sensoren an einem vorteilhaften Messbereich angeordnet werden können. Darüber hinaus können die Messeinrichtungen 32 bzw. Sensoren ausschließlich oder zusätzlich hydrodynamische und/oder aerodynamische Strukturen aufweisen, um den Mediumfluss in ihren jeweiligen Messbereichen im Inneren des Strömungskörpers 12 zu beeinflussen. Selbiges gilt für den Leitungsteilkörper 122.

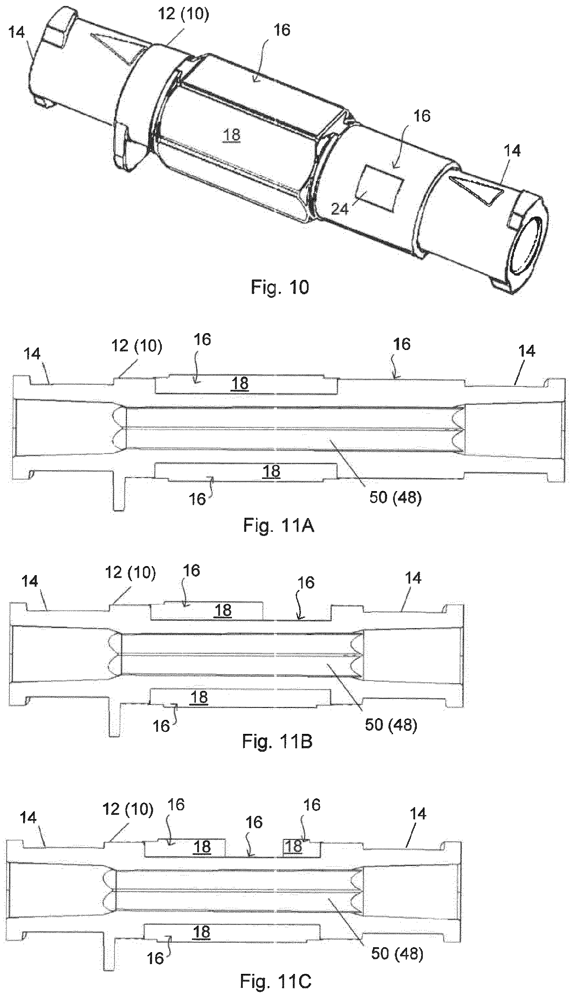

**Figur 10** zeigt eine weitere beispielhafte Ausführungsform der Erfindung mit zwei Koppeleinrichtungen 16. Bevorzugt weisen der Leitungskörper 12 und die Anschlussbereiche 14 ein erstes Material, beispielsweise ein Thermoplast, wie z.B. Polybutylenterephthalat (PBT/PTMT), Celanex^{®} oder Vestodur^{®}, auf.

Eine erste Koppeleinrichtung 16 weist in der gezeigten Ausführungsform eine Beschichtung 18 auf, welche ein zweites Material auf, welches von dem ersten Material abweicht. Vorteilhafterweise weist das zweite Material ein thermoplastisches Elastomer auf, z.B. umfassend bzw. im wesentlichen bestehend aus TPE (Thermoplastisches Elastomer wie z.B. TPA, TPC, TPO, TPS, TPU, TPV), Silikon, NBR (Acrylnitril-Butadien-Kautschuk) oder Materialien mit ähnlichen Eigenschaften. Eine solche oder ähnliche Beschichtung ermöglicht insbesondere eine vorteilhafte mechanische Kopplung einer Messeinrichtung, beispielsweise eines Temperatursensor, mit der ersten Koppeleinrichtung 16.

Wie in Figur 10 gezeigt weist die erste Koppeleinrichtung 16 einen vieleckigen, z.B. sechseckigen, Querschnitt bzw. Außenform auf. Das zweite Material kann dabei über den gesamten Außenumfang oder über Teilbereiche davon vorgesehen sein. Ebenso kann der Außenumfang der Koppeleinrichtung 16 auch eine im Wesentlichen runde Form aufweisen.

Die zweite Koppeleinrichtung 16 des in der Figur 10 gezeigten Leitungskörpers 12 umfasst beispielsweise einen im Wesentlichen runden Querschnitt sowie ein optisches Fenster 24, z.B. in der Form eines Glassubstrats bzw. einer Glasscheibe oder eines im optischen Bereich des elektromagnetischen Spektrums im Wesentlichen transparenten Körpers aus z.B. Saphir, Quarz, MgF2, CaF2, Ge, Si, Diamant. Anstelle oder zusätzlich zu der gezeigten Ausführung der zweiten Koppeleinrichtung 16 können auch andere Ausgestaltungen der in dieser Anmeldung offenbarten Koppeleinrichtungen 16 vorgesehen sein, insbesondere eine Koppeleinrichtung 16 mit Metallplättchen 22, Membran 26 oder Elektrode(n) 28.

**Figur 11A** zeigt einen Längsschnitt eines Leitungskörpers 12 gemäß der Figur 10. Die Beschichtung 18 der ersten Koppeleinrichtung 16 ist in der gezeigten, besonders bevorzugten Ausführungsform in eine Aussparung im Leitungskörper 12 eingebettet. Parameter der Beschichtung 18 können je nach Anforderung variieren - insbesondere Dicke, Schichtanzahl, Schichtmaterial(ien), Länge, Breite und/oder Oberflächenbeschaffenheit. Der gezeigte Leitungskörper 12 weist darüber hinaus eine strömungsbildverändernde Geometrie 50 im Mediumströmungsbereich 48 der ein oder mehreren Koppeleinrichtungen 16 auf (wie zu Figur 9 beschrieben). Wie in Figur 11A gezeigt kann der Leitungskörper 12 optional eine weitere, dritte Koppeleinrichtung 16 beispielsweise auf der gegenüberliegenden Seite der ersten Koppeleinrichtung 16 aufweisen. Mit den abgebildeten ersten und dritten Koppeleinrichtung 16 kann beispielweise eine besonders vorteilhafte Redundanzmessung mit zwei Messeinrichtung 32 selben Typs an selber Stelle im Mediumströmungsbereich 48 erfolgen.

**Figur 11B** zeigt einen Längsschnitt einer alternativen Ausführung des in den Figuren 10 und 11A gezeigten Leitungskörpers 12. Die vorliegende beispielhafte Ausführungsform unterscheidet sich insbesondere in der zweiten Koppeleinrichtung 16, welche in einem Bereich des Leitungskörpers 12 mit reduziertem Durchmesser angeordnet ist. Die zweite Koppeleinrichtung 16 befindet sich in diesem Beispiel in einer Aussparung der Beschichtung 18 der ersten Koppeleinrichtung 16 wie in den Figuren 10 und 11A gezeigt und ist insbesondere für die Kopplung eines Temperatursensors geeignet. Die dargestellte Aussparung in der Beschichtung 18 kann beispielsweise durch Auslassen dieses Bereichs beim Aufbringen einer Beschichtung 18 oder durch (zumindest teilweise) Entfernen einer zuvor aufgebrachten Beschichtung 18 erfolgen. Eine solche Ausführung zeichnet sich insbesondere durch eine kompakte Bauweise und vereinfachte Fertigung aus. Darüber hinaus kann durch entsprechende Eigenschaften des zweiten Materials (z.B. temperaturisolierend/schwach temperaturleitend) eine störende Beeinflussung einer Temperaturmessung verhindert, zumindest aber deutlich reduziert werden, Die zweite Koppeleinrichtung 16 kann darüber hinaus eine Beschichtung mit einem dritten Material und/oder eine andere Form wie die erste Koppeleinrichtung 16 aufweisen.

**Figur 11C** zeigt eine weitere alternative Ausführungsform, bei der die zweite Koppeleinrichtung 16 zwischen der ersten und einer optionalen weiteren, vierten Koppeleinrichtung 16 vorgesehen ist, wobei die vierte Koppeleinrichtung 16 bevorzugt identischen zu der ersten Koppeleinrichtung 16 ist. Auf diese Weise kann eine weitere Messeinrichtung 32 mit dem Leitungskörper 12 gekoppelt werden - mit Beibehalten der kompakten Abmessung des Leitungskörpers 12.

Die Leitungskörper 12 der Figuren 11B und 11C weisen ebenfalls bevorzugt eine strömungsbildverändernde Geometrie 50 im Mediumströmungsbereich 48 auf. Darüber hinaus kann der Leitungskörper 12 der Figuren 10-11C alternativ oder zusätzlich weitere identisch und/oder anders ausgestaltete Koppeleinrichtungen 16 sowie weitere Merkmale der in dieser Anmeldung beschriebenen Ausführungsformen aufweisen.

### Bezugszeichenliste

- 1: Behälter (z.B. Bioreaktor)
- 4: Medium
- 6: Ventil
- 8: Rührvorrichtung
- 10: Leitungssystem
- 12: Leitungskörper
- 14: Anschlussbereich
- 16: Koppeleinrichtung
- 18: Beschichtung
- 20: Empfangseinrichtung
- 22: Metallplättchen
- 24: Fenster
- 26: Membran
- 28: Elektrode
- 30: Messsystem
- 32: Messeinrichtung
- 36: Befestigungsvorrichtung
- 38: Drucksensor
- 40: Durchflusssensor
- 42: Leitfähigkeitssensor
- 42A: Frontplatte
- 42B: Rückplatte
- 43: Federbeaufschlagter Kontakt
- 44: Temperatursensor
- 46: pH-Sensor
- 48: Mediumströmungsbereich
- 50: strömungsbildverändernde Geometrie
- 52: Aussparung
- 112: Leitungshauptkörper
- 114: Ausschnitt
- 122: Leitungsteilkörper

## Patentansprüche

1. Behälter (1) zur Aufbewahrung, Mischung und/oder Kultivierung eines Mediums, insbesondere ein Bioreaktor (1) für ein Medium, mit einem Leitungssystem (10) als Ableitung, Zuleitung und/oder Bypassleitung des Behälters (1), wobei das Leitungssystem (10) zumindest umfasst:
einen Leitungskörper (12), welcher einstückig ausgebildet und geeignet ist, von einem Medium durchströmt zu werden, wobei der Leitungskörper (12) aufweist:
einen ersten und einen zweiten Anschlussbereich (14) zum Anschließen insbesondere an den Behälter (1) und/oder das Leitungssystem (10); und
wenigstens eine erste und eine zweite Koppeleinrichtung (16) im Bereich zwischen den Anschlussbereichen (14), wobei die erste und zweite Koppeleinrichtung (16) strukturell unterschiedlich ausgestaltet und dazu ausgebildet sind, um mit jeweils strukturell unterschiedlichen Messeinrichtungen (32) gekoppelt zu werden.

2. Behälter (1) nach Anspruch 1, wobei der Leitungskörper (12) zumindest eine dritte Koppeleinrichtung (16) aufweist und, bevorzugt, wobei die erste, zweite und dritte Koppeleinrichtung (16) strukturell unterschiedlich ausgestaltet sind.

3. Behälter (1) nach einem der vorhergehenden Ansprüche, wobei die Koppeleinrichtungen (16) in Umfangsrichtung des Leitungskörpers (12) und/oder in Strömungsrichtung eines durch den Leitungskörper (12) strömenden Mediums voneinander beabstandet angeordnet sind.

4. Behälter (1) nach einem der vorhergehenden Ansprüche, wobei die mit den Koppeleinrichtungen (16) des Leitungskörpers (12) koppelbaren Messeinrichtungen insbesondere dafür geeignet sind, Temperatur, Druck, Durchflussrate, Sauerstoffgehalt, pH-Wert, Leitfähigkeit, Viskosität und/oder optische Parameter eines durch den Leitungskörper (12) strömenden Mediums zu messen; und, vorzugsweise,/oder
wobei der Leitungskörper (12) zur einmaligen Verwendung bestimmt ist bzw. ein Einweg-Leitungskörper ist.

5. Leitungskörper (12) für ein Leitungssystem (10) zum Ableiten, Zuleiten und/oder Umleiten eines Mediums eines Behälters (1) zur Aufbewahrung, Mischung und/oder Kultivierung eines Mediums, insbesondere eines Bioreaktors, wobei der Leitungskörper (12) einstückig ausgebildet ist und aufweist:
einen ersten und einen zweiten Anschlussbereich (14) zum Anschließen des Leitungskörpers (12) insbesondere an einen Behälter (1) und/oder an das Leitungssystem (10); und
wenigstens eine erste und eine zweite Koppeleinrichtung (16) im Bereich zwischen den Anschlussbereichen (14), wobei die erste und zweite Koppeleinrichtung (16) strukturell unterschiedlich ausgestaltet und dazu ausgebildet sind, um mit jeweils strukturell unterschiedlichen Messeinrichtungen (32) gekoppelt zu werden.

6. Leitungskörper (12) nach Anspruch 5, wobei der Leitungskörper (12) zumindest eine dritte Koppeleinrichtung (16) aufweist und, bevorzugt, wobei die erste, zweite und dritte Koppeleinrichtung (16) des Leitungskörpers (12) strukturell unterschiedlich ausgestaltet sind; und, vorzugsweise,/oder
wobei die mit den Koppeleinrichtungen (16) des Leitungskörpers (12) koppelbaren Messeinrichtungen (32) insbesondere dafür geeignet sind, Temperatur, Druck, Durchflussrate, Sauerstoffgehalt, pH-Wert, Leitfähigkeit, Viskosität und/oder optische Parameter eines durch den Leitungskörper (12) strömenden Mediums zu messen.

7. Leitungskörper (12) nach einem der Ansprüche 5 oder 6, wobei der Leitungskörper (12) zur einmaligen Verwendung bestimmt ist bzw. ein Einweg-Leitungskörper ist.

8. Leitungskörper (12) nach einem der Ansprüche 5 bis 7, wobei zumindest ein Bereich einer Koppeleinrichtung (16) des Leitungskörpers (12) aufweist:
eine Wandstärke von weniger als etwa 3 mm, bevorzugt weniger als etwa 2 mm; und/oder
eine Beschichtung (18) auf der Außenseite des Leitungskörpers (12);
und/oder
eine Wärmeleitfähigkeit, um Messungen der Temperatur des Mediums von außerhalb des Leitungskörpers (12) zu ermöglichen; und/oder
eine optische Durchlässigkeit, um Messungen optischer Parameter des Mediums von außerhalb des Leitungskörpers (12) zu ermöglichen; und/oder
eine Schalldurchlässigkeit, um Messungen der Durchflussrate des Mediums von außerhalb des Leitungskörpers (12) zu ermöglichen.

9. Leitungskörper (12) nach einem der Ansprüche 5 bis 8, wobei eine Koppeleinrichtung (14) des Leitungskörpers (12) ein oder mehrere Öffnungen in dem Leitungskörper (12) zum Empfangen zumindest eines Teils einer Messeinrichtung (32) bzw. zum Einführen zumindest eines Teils einer Messeinrichtung (32) in das Innere des Leitungskörpers (12) aufweist; und, vorzugsweise,/oder
wobei eine Koppeleinrichtung (14) des Leitungskörpers (12) eine auslenkbare Membran (26) aufweist, um Messungen des Drucks innerhalb des Leitungskörpers (12) bzw. des Mediums von außerhalb des Leitungskörpers (12) zu ermöglichen, wobei die auslenkbare Membran (16) bevorzugt aufweist:
eine erste Seite, die dem Inneren des Leitungskörpers (12) zugewandt ist und mit dem Medium im Inneren des Leitungskörpers (12) in Kontakt treten kann; und
eine zweite Seite, die von außerhalb des Leitungskörpers (12) zugänglich ist.

10. Leitungskörper (12) einem der Ansprüche 5 bis 9, wobei eine Koppeleinrichtung (16) des Leitungskörpers (12) zwei oder mehr Elektroden (28) aufweist und wobei:
eine erste Seite der zwei oder mehr Elektroden (28) dem Inneren des Leitungskörpers (12) zugewandt ist und mit dem Medium im Inneren des Leitungskörpers (12) in Kontakt treten kann; und
eine zweite Seite der zwei oder mehr Elektroden (28) von außerhalb des Leitungskörpers (12) zugänglich ist.

11. Leitungskörper (12) nach einem der Ansprüche 5 bis 10, wobei eine Koppeleinrichtung (16) eine Empfangseinrichtung (20), insbesondere einen Schacht und/oder eine Schiene, zum Empfangen zumindest eines Teils einer Messeinrichtung (32) aufweist.

12. Leitungskörper (12) nach einem der Ansprüche 5 bis 11, wobei der Leitungskörper (12) für eine Bestrahlungs- und/oder Autoklav- bzw. Dampfsterilisation geeignet ist; und, vorzugsweise,/oder
wobei Koppeleinrichtungen (16) in Strömungsrichtung eines durch den Leitungskörper (12) strömenden Mediums und/oder in Umfangsrichtung des Leitungskörpers (12) voneinander beabstandet angeordnet sind.

13. Leitungskörper (12) nach einem der Ansprüche 5 bis 12, weiter umfassend eine strömungsbildverändernde Geometrie (50) im Mediumströmungsbereich (48) des Leitungskörpers (12) zum Beeinflussen der Strömungseigenschaften des Mediums.

14. Leitungskörper (12) nach einem der Ansprüche 5 bis 13, wobei der Leitungskörper (12) umfasst:
einen Leitungshauptkörper (112) mit den Anschlussbereichen (14) und einem Ausschnitt (114), und
einem Leitungsteilkörper (122) mit zumindest zwei Koppeleinrichtungen (16),
wobei der Ausschnitt (114) des Leitungshauptkörpers (112) dazu ausgebildet ist, den Leitungsteilkörper (122) zumindest bereichsweise zu empfangen.

15. Messsystem (30) für einen Behälter (1) zur Aufbewahrung, Mischung und/oder Kultivierung eines Mediums, insbesondere einen Bioreaktor, zum Messen von Parametern eines Mediums umfassend:
einen Leitungskörper (12) gemäß einem der Ansprüche 5 bis 14; und
zwei oder mehr Messeinrichtungen (32), welche jeweils mit einer der Koppeleinrichtungen (16) des Leitungskörpers (12) gekoppelt sind und wobei zumindest zwei der Messeinrichtungen (32) strukturell unterschiedlich ausgebildet sind.

## Claims

1. Container (1) for storing, mixing and/or cultivating a medium, in particular a bioreactor (1) for a medium, with a conduit system (10) as a discharge line, feed line and/or bypass line of the container (1), the conduit system (10) comprising at least:
a conduit body (12) which is formed in one piece and is suitable for a medium to flow through it, wherein the conduit body (12) has:
a first and a second connection region (14) for connecting in particular to the container (1) and/or the conduit system (10); and
at least a first and a second coupling device (16) in the region between the connection regions (14), wherein the first and second coupling devices (16) are structurally differently configured and are designed to be coupled to respectively structurally different measuring devices (32).

2. Container (1) according to claim 1, wherein the conduit body (12) has at least a third coupling device (16) and, preferably, the first, second and third coupling devices (16) are structurally differently configured.

3. Container (1) according to one of the preceding claims, wherein the coupling devices (16) are arranged spaced apart from one another in the circumferential direction of the conduit body (12) and/or in the flow direction of a medium flowing through the conduit body (12).

4. Container (1) according to one of the preceding claims, wherein the measuring devices couplable to the coupling devices (16) of the conduit body (12) are in particular suitable for measuring temperature, pressure, flow rate, oxygen content, pH value, conductivity, viscosity and/or optical parameters of a medium flowing through the conduit body (12); and, preferably,/or
the conduit body (12) is intended for single use or is a single-use conduit body.

5. Conduit body (12) for a conduit system (10) for discharging, feeding, diverting and/or rerouting a medium of a container (1) for storing, mixing and/or cultivating a medium, in particular a bioreactor, wherein the conduit body (12) is formed in one piece and has:
a first and a second connection region (14) for connecting the conduit body (12) in particular to a container (1) and/or to the conduit system (10); and
at least a first and a second coupling device (16) in the region between the connection regions (14), wherein the first and second coupling devices (16) are structurally differently configured and are designed to be coupled to respectively structurally different measuring devices (32).

6. Conduit body (12) according to claim 5, wherein the conduit body (12) has at least a third coupling device (16) and, preferably, the first, second and third coupling devices (16) of the conduit body (12) are structurally differently configured; and, preferably,/or
the measuring devices (32) couplable to the coupling devices (16) of the conduit body (12) are in particular suitable for measuring temperature, pressure, flow rate, oxygen content, pH value, conductivity, viscosity and/or optical parameters of a medium flowing through the conduit body (12).

7. Conduit body (12) according to one of claims 5 or 6, wherein the conduit body (12) is intended for single use or is a single-use conduit body.

8. Conduit body (12) according to one of claims 5 to 7, wherein at least a region of a coupling device (16) of the conduit body (12) has:
a wall thickness of less than approximately 3 mm, preferably less than approximately 2 mm; and/or
a coating (18) on the outer side of the conduit body (12); and/or
a thermal conductivity, to enable measurements of the temperature of the medium from outside the conduit body (12); and/or
an optical transparency, to enable measurements of optical parameters of the medium from outside the conduit body (12); and/or
an acoustic transparency, to enable measurements of the flow rate of the medium from outside the conduit body (12).

9. Conduit body (12) according to one of claims 5 to 8, wherein a coupling device (14) of the conduit body (12) has one or more openings in the conduit body (12) for receiving at least a part of a measuring device (32) or for introducing at least a part of a measuring device (32) into the interior of the conduit body (12); and, preferably,/or
a coupling device (14) of the conduit body (12) has a deflectable membrane (26) to enable measurements of the pressure inside the conduit body (12) or of the medium from outside the conduit body (12), wherein the deflectable membrane (16) preferably has:
a first side which faces the interior of the conduit body (12) and can come into contact with the medium inside the conduit body (12); and
a second side which is accessible from outside the conduit body (12).

10. Conduit body (12) according to one of claims 5 to 9, wherein a coupling device (16) of the conduit body (12) has two or more electrodes (28) and wherein:
a first side of the two or more electrodes (28) faces the interior of the conduit body (12) and can come into contact with the medium inside the conduit body (12); and
a second side of the two or more electrodes (28) is accessible from outside the conduit body (12).

11. Conduit body (12) according to one of claims 5 to 10, wherein a coupling device (16) has a receiving device (20), in particular a shaft and/or a rail, for receiving at least a part of a measuring device (32).

12. Conduit body (12) according to one of claims 5 to 11, wherein the conduit body (12) is suitable for irradiation and/or autoclave or steam sterilization; and, preferably,/or
the coupling devices (16) are arranged spaced apart from one another in the flow direction of a medium flowing through the conduit body (12) and/or in the circumferential direction of the conduit body (12).

13. Conduit body (12) according to one of claims 5 to 12, further comprising a flow-image-altering geometry (50) in the medium flow region (48) of the conduit body (12) for influencing the flow properties of the medium.

14. Conduit body (12) according to one of claims 5 to 13, wherein the conduit body (12) comprises:
a main conduit body (112) with the connection regions (14) and a cutout (114), and
a partial conduit body (122) with at least two coupling devices (16),
wherein the cutout (114) of the main conduit body (112) is designed to receive the partial conduit body (122) at least in regions.

15. Measuring system (30) for a container (1) for storing, mixing and/or cultivating a medium, in particular a bioreactor, for measuring parameters of a medium, comprising:
a conduit body (12) according to one of claims 5 to 14; and
two or more measuring devices (32) which are respectively coupled to one of the coupling devices (16) of the conduit body (12) and of which at least two of the measuring devices (32) are structurally differently configured.

## Revendications

1. Récipient (1) pour le stockage, le mélange et/ou la culture d'un milieu, en particulier un bioréacteur (1) pour un milieu, avec un système de conduits (10) en tant que conduit d'évacuation, conduit d'alimentation et/ou conduit de dérivation du récipient (1), le système de conduits (10) comprenant au moins :
un corps de conduit (12), lequel est réalisé d'une seule pièce et est adapté pour être traversé par un milieu, le corps de conduit (12) présentant :
un premier et un deuxième domaine de raccordement (14) pour le raccordement en particulier au récipient (1) et/ou au système de conduits (10) ; et
au moins un premier et un deuxième dispositif de couplage (16) dans le domaine entre les domaines de raccordement (14), les premier et deuxième dispositifs de couplage (16) étant structurellement différemment conformés et étant conçus pour être couplés à des dispositifs de mesure (32) respectivement structurellement différents.

2. Récipient (1) selon la revendication 1, dans lequel le corps de conduit (12) présente au moins un troisième dispositif de couplage (16) et, de préférence, les premier, deuxième et troisième dispositifs de couplage (16) sont structurellement différemment conformés.

3. Récipient (1) selon l'une des revendications précédentes, dans lequel les dispositifs de couplage (16) sont disposés de manière espacée les uns des autres dans la direction circonférentielle du corps de conduit (12) et/ou dans la direction d'écoulement d'un milieu s'écoulant à travers le corps de conduit (12).

4. Récipient (1) selon l'une des revendications précédentes, dans lequel les dispositifs de mesure couplables aux dispositifs de couplage (16) du corps de conduit (12) sont en particulier adaptés pour mesurer la température, la pression, le débit, la teneur en oxygène, la valeur de pH, la conductivité, la viscosité et/ou des paramètres optiques d'un milieu s'écoulant à travers le corps de conduit (12) ; et, de préférence,/ou
le corps de conduit (12) est destiné à un usage unique ou est un corps de conduit à usage unique.

5. Corps de conduit (12) pour un système de conduits (10) destiné à l'évacuation, l'alimentation, la dérivation et/ou la déviation d'un milieu d'un récipient (1) pour le stockage, le mélange et/ou la culture d'un milieu, en particulier d'un bioréacteur, le corps de conduit (12) étant réalisé d'une seule pièce et présentant :
un premier et un deuxième domaine de raccordement (14) pour le raccordement du corps de conduit (12) en particulier à un récipient (1) et/ou au système de conduits (10) ; et
au moins un premier et un deuxième dispositif de couplage (16) dans le domaine entre les domaines de raccordement (14), les premier et deuxième dispositifs de couplage (16) étant structurellement différemment conformés et étant conçus pour être couplés à des dispositifs de mesure (32) respectivement structurellement différents.

6. Corps de conduit (12) selon la revendication 5, dans lequel le corps de conduit (12) présente au moins un troisième dispositif de couplage (16) et, de préférence, les premier, deuxième et troisième dispositifs de couplage (16) du corps de conduit (12) sont structurellement différemment conformés ; et, de préférence,/ou
les dispositifs de mesure (32) couplables aux dispositifs de couplage (16) du corps de conduit (12) sont en particulier adaptés pour mesurer la température, la pression, le débit, la teneur en oxygène, la valeur de pH, la conductivité, la viscosité et/ou des paramètres optiques d'un milieu s'écoulant à travers le corps de conduit (12).

7. Corps de conduit (12) selon l'une des revendications 5 ou 6, dans lequel le corps de conduit (12) est destiné à un usage unique ou est un corps de conduit à usage unique.

8. Corps de conduit (12) selon l'une des revendications 5 à 7, dans lequel au moins un domaine d'un dispositif de couplage (16) du corps de conduit (12) présente :
une épaisseur de paroi inférieure à environ 3 mm, de préférence inférieure à environ 2 mm ; et/ou
un revêtement (18) sur la face extérieure du corps de conduit (12) ; et/ou
une conductivité thermique, pour permettre des mesures de la température du milieu depuis l'extérieur du corps de conduit (12) ; et/ou
une transparence optique, pour permettre des mesures de paramètres optiques du milieu depuis l'extérieur du corps de conduit (12) ; et/ou
une transparence acoustique, pour permettre des mesures du débit du milieu depuis l'extérieur du corps de conduit (12).

9. Corps de conduit (12) selon l'une des revendications 5 à 8, dans lequel un dispositif de couplage (14) du corps de conduit (12) présente une ou plusieurs ouvertures dans le corps de conduit (12) pour recevoir au moins une partie d'un dispositif de mesure (32) ou pour introduire au moins une partie d'un dispositif de mesure (32) dans l'intérieur du corps de conduit (12) ; et, de préférence,/ou
un dispositif de couplage (14) du corps de conduit (12) présente une membrane déformable (26) pour permettre des mesures de la pression à l'intérieur du corps de conduit (12) ou du milieu depuis l'extérieur du corps de conduit (12), la membrane déformable (16) présentant de préférence :
un premier côté, qui est tourné vers l'intérieur du corps de conduit (12) et peut entrer en contact avec le milieu à l'intérieur du corps de conduit (12) ; et
un deuxième côté, qui est accessible depuis l'extérieur du corps de conduit (12).

10. Corps de conduit (12) selon l'une des revendications 5 à 9, dans lequel un dispositif de couplage (16) du corps de conduit (12) présente deux ou plus électrodes (28) et dans lequel :
un premier côté des deux ou plus électrodes (28) est tourné vers l'intérieur du corps de conduit (12) et peut entrer en contact avec le milieu à l'intérieur du corps de conduit (12) ; et
un deuxième côté des deux ou plus électrodes (28) est accessible depuis l'extérieur du corps de conduit (12).

11. Corps de conduit (12) selon l'une des revendications 5 à 10, dans lequel un dispositif de couplage (16) présente un dispositif de réception (20), en particulier un puits et/ou un rail, pour recevoir au moins une partie d'un dispositif de mesure (32).

12. Corps de conduit (12) selon l'une des revendications 5 à 11, dans lequel le corps de conduit (12) est adapté pour une stérilisation par irradiation et/ou par autoclave ou par vapeur ; et, de préférence,/ou
les dispositifs de couplage (16) sont disposés de manière espacée les uns des autres dans la direction d'écoulement d'un milieu s'écoulant à travers le corps de conduit (12) et/ou dans la direction circonférentielle du corps de conduit (12).

13. Corps de conduit (12) selon l'une des revendications 5 à 12, comprenant en outre une géométrie modifiant l'image d'écoulement (50) dans le domaine d'écoulement du milieu (48) du corps de conduit (12) pour influencer les propriétés d'écoulement du milieu.

14. Corps de conduit (12) selon l'une des revendications 5 à 13, dans lequel le corps de conduit (12) comprend :
un corps de conduit principal (112) avec les domaines de raccordement (14) et un évidement (114), et
un corps de conduit partiel (122) avec au moins deux dispositifs de couplage (16),
l'évidement (114) du corps de conduit principal (112) étant conçu pour recevoir le corps de conduit partiel (122) au moins par zones.

15. Système de mesure (30) pour un récipient (1) pour le stockage, le mélange et/ou la culture d'un milieu, en particulier un bioréacteur, pour mesurer des paramètres d'un milieu, comprenant :
un corps de conduit (12) selon l'une des revendications 5 à 14 ; et
deux ou plus dispositifs de mesure (32), lesquels sont respectivement couplés à l'un des dispositifs de couplage (16) du corps de conduit (12) et parmi lesquels au moins deux des dispositifs de mesure (32) sont structurellement différemment conformés.
